# EUROPEAN PATENT SPECIFICATION

(11) **EP 1 299 558 B1**
(45) Date of publication and mention of the grant of the patent: **10.05.2006**
(21) Application number: 01947681.1
(22) Date of filing: 09.07.2001
(51) Int. Cl.: C12Q 1/00

(54) **SCREENING METHOD**
SCREENING-VERFAHREN
PROCEDE DE CRIBLAGE

(30) Priority: 07.07.2000 GB 0016692; 08.12.2000 US 254459 P
(43) Date of publication of application: 09.04.2003
(73) Proprietor: Eirx Therapeutics Ltd, Cork (IE)
(72) Inventor: COTTER, Tom, EiRx Therapeutics Ltd, Cork (IE); HAYES, Ian, EiRx Therapeutics Ltd, Cork (IE); MURPHY, Finbarr, EiRx Therapeutics Ltd, Cork (IE); SEERY, Liam, EiRx Therapeutics Ltd, Cork (IE)
(74) Representative: Furlong, Isla Jane
(86) International application number: PCT/GB2001/003101
(87) International publication number: WO 2002/004657

(56) References cited:
- EP-A- 0 926 242
- WO-A-99/32514
- Y. WANG, T. REA, J. BIAN, S.GRAY, Y. SUN: "Identification of genes responsive to etoposide-induced apoptosis: application of DNA chip technology" FEBS, vol. 445, 1999, pages 269-273, XP002209541
- W. YUGANG, L. HONTAO, Z. YINGMEI, M. DALONG: "cDNA cloning and expresion of an apoptosis-related gene, human TFAR15 gene" SCIENCE IN CHINA SERIES C LIFE SCIENCES, vol. 42, no. 3, 1999, pages 323-329, XP002209542
- COXON ANGELA ET AL: "Cytokine-activated endothelial cells delay neutrophil apoptosis in vitro and in vivo: A role for granulocyte/macrophage colony-stimulating factor" JOURNAL OF EXPERIMENTAL MEDICINE, TOKYO, JP, vol. 190, no. 7, 4 October 1999 (1999-10-04), pages 923-933, XP002194526 ISSN: 0022-1007
- S.J. WILSON, B.A. LEONE, D. ANDERSON, A. MANNING, S.T. HOLGATE: "Immunohistochemical analysis of the activation of NF-kappaB and expression of associated cytokines and adhesion molecules in human models of allergic inflammation" JOURNAL OF PATHOLOGY, vol. 189, 1999, pages 265-272, XP002209544
- P. WEINMANN, P. GAEHTGENS, B. WALZOG: "Bcl-X1 and Bax-alpha mediated regulation of Apoptosis of human neutrophils via caspase-3" BLOOD, vol. 93, no. 9, 1999, pages 3106-3115, XP002209559
- MCCUBREY J.A:, MAY W. STRATFORD, DURINO V. MUFSON A.: "serine/threonine phosphorylation in cytokine signal transduction" LEUKEMIA, vol. 14, no. 1, January 2000 (2000-01), pages 9-21, XP002209543

## Description

The present invention relates to a method for identifying genes, which are involved in the modulation of apoptosis in cells. In particular, the invention relates to a novel method for identifying and characterising those genes, and the molecular mechanisms involved in the GM-CSF mediated inhibition of cell death.

Programmed cell death or apoptosis is a genetically programmed process by which cells die under both physiological and a variety of pathological conditions (Kerr et al, Br. J. Cancer, 26, 239-257, 1972). It serves as the counter-balancing force to mitosis during adult life and is a major contributor to the sculpting of physiological structures during the many processes of development (Wyllie et al, Int. Rev. Cytol, 68, 251-305, 1980). It is characterised by a number of well-defined biochemical hallmarks. These include DNA fragmentation, caused by the activation of an endogenous endonuclease enzyme (Wyllie, Nature, 284, 555-556,1980; Enari et al., Nature, 391, 43-50, 1998). The result is a DNA ladder pattern that can be readily visualised in agarose cells. Coupled with DNA fragmentation is cell shrinkage (Wesselbory et al., Cell Immunol. 148, 234-41, 1993) where water is actively extruded from the cell. The apoptotic cell then undergoes fragmentation into apoptotic bodies that are engulfed by neighbouring cells or cells of the reticuloedothelial system.

The number of factors which are know to induce survival in particular cell types is ever increasing (e.g. IL2, IL3, IL4, IL5, IL8, GM-CSF, insulin like growth factor 1, NGF, VEGF, PDGF, SCF, LIF, EGF etc.). Many of these survival factors appear to share a commonality in the survival pathway (Datta SR et al. *Genes and Development* 13: 2905-2927, 1999). For an extracellular stimuli, to confer survival on a cell, it must inhibit the endogenous apoptotic machinery. The model predicts that there is a series of temporal events that occur upon survival factor/ receptor interaction. The first of these is tyrosine phosphorylation at the plasma membrane due either to intrinsic receptor tyrosine kinase activity (e.g. the insulin growth factor 1 receptor), or indirectly coupled to tyrosine kinases or alternatively directly coupled to several transmembrane G protein-coupled receptors.

Blood neutrophils have relatively short lives with greater than 80% of them apoptosing within the first 24 hours. Apoptotic neutrophils are phagocytosed by macrophages via thrombospondin and macrophage CD36/ vitronectin receptor (Savil 1992, Clinical Science 83, 649-55, Savil et al. 1993, Immunology Today 14,131-136) and thus prevent release of potentially lethal cocktail of enzymes in the host, should the neutrophil undergo necrosis. However, certain inflammatory environments favour the survival of neutrophils. In vitro, several cytokines including GM-CSF, IL-1, IL-2, IL-8 and IFNγ can delay neutrophil apoptosis (Brach et al, 1992, Blood 80, 2920 -2924; Calotta et al 1992, Blood 80, 2012-2020, Lee et al 1993, J Leuk Biol 54, 283 - 388, Pericle et al 1994, Eur. J. Immuno124, 440 - 444, Get ref for IL8). Furthermore, inflammatory proteins e.g. C5A and bacterial products e.g. LPS) have also been shown to inhibit apoptosis. These findings together with other results demonstrating that the presence of either actinomycin D or cycloheximide can promote apoptosis in PMN (Whyte et al, 1991, Clin Sci. 80:5p) suggests a role for active gene expression and translation in control of PMN apoptosis. Moreover, other investigators have shown that NKκB regulated genes seem to play a critical role in preventing apoptosis induced by TNFα, since inhibition of this transcription factor using the fungal metabolite Gliotoxin, induces rapid apoptosis (Ward et al. 1999, J. Biol. Chem. 274. 4309-4318). The same investigators also demonstrated that blocking NFκB with Gliotoxin removes the anti-apoptotic effect of LPS. Yoshida et al have identified an alternative mode of action of gliotoxin. These investigators demonstrated that gliotoxin inhibited NADPH oxidase and consequently prevented the onset of superoxide generation by human neutrophils in response to phorbol myristate. (Yoshida et al Biochem Biophys Res Commun 2000,268(3) 716-23).

Granulocyte macrophage colony-stimulating factor (GM-CSF) is known to inhibit PMN apoptosis both in vitro and in vivo (Cox et al. 1992, Am. J. Respiratory Cell Mol Biol. 7, 507; Chintinis et al 1996, J. Leuk Biol 59:835). One consequence of GM-CSF treatment of PMN is a time and dose dependent tyrosine phosphorylation event within the cell (McCall et al., 1991, Blood 78(7) 1842-52). That tyrosine phosphorylation is implicated in the regulation of apoptosis has been demonstrated (Simon et al 1995, Int. Arch Allergy Immunol. 107, 338-339). These workers demonstrated that the effect of GM-CSF on granulocyte cell death could be attenuated by the tyrosine kinase inhibitor genestein, suggesting that increases in tyrosine phosphorylation are essential to inhibit cell death. To further analyse a role for tyrosine phosphorylation, the authors increased levels of tyrosine phosphorylation using the protein- phosphatase inhibitor phenylansine oxide (PAO). Similar to GM-CSF, treatment of the cells with PAO is followed by a large increase in tyrosine phosphorylation and matched inhibition of apoptosis. Inhibitors of tyrosine phosphorylation (Genestein and Herbimycin A) reversed the effects of PAO on tyrosine phosphorylation and neutrophil apoptosis.

Furthermore, Wei et al (J. Immunology 1996,157, 5155-5162) suggested specificity in the anti-apoptotic signalling pathway by showing that GM-CSF inhibition of programmed cell death did not appear to be related to known proteins associated with cell survival i.e. p53, cdc2, Rb, and Bcl-2. However GM-CSF did induce a rapid activation of Lyn, a src family tyrosine kinase, and Lyn antisense treatment of neutrophils reversed the survival promoting effect of GM-CSF. Other investigators have demonstrated that GM-CSF selectively induced tyrosine phosphorylation of Extracellular Signal-Related kinase (ERK), a member of microtubule associated protein kinase (MAPK) family (Yuo et al. 1997, BBRC 235, 42- 46). Al-Shami et al. (Blood 1997, 89(3) 1035-1044) has shown that GM-CSF induces both a time and concentration- dependent increase in the level of tyrosine phosphorylation of the PI-3-kinase regulatory subunit p85, possibly via lyn kinase. In corroboration of these results, Klein et al. (J. Immunol. 2000, 164, 4286-4291), using pharmacological inhibitors of signal transduction, further demonstrated a role for PI 3-kinase and ERK. These investigators showed that GM-CSF caused a rapid phosphorylation of the protein Akt, a substrate for PI 3-kinase. Akt phosphorylation is in turn associated with phosphorylation of BAD, a pro-apoptotic member of the Bcl-2 family. The authors hypothesised that this phosphorylation resulted in disengagement of Bad with anti-apoptotic family members of Bcl-2 family, allowing them to prevent neutrophil apoptosis.

The link between GM-CSF and tyrosine phosphorylation and inhibition of programmed cell death is presently unknown. Previously, prolonged survival of PMN caused by inhibition of apoptosis is observed in bcl-2 transgenic mice (Lagasse and Weissman, 1994, J. Exp. Med. 179 1047). This result is surprising since normal peripheral blood neutrophils are negative for bcl-2 (Wei et al. J. Immunology 1996,157, 5155-5162), however it does show that targets for the Bcl-2 family of apoptosis associated proteins can control PMN apoptosis. Weinnman et al. (1999, Blood, 93, 3106-3115) investigated the role of other members of the Bcl-2 family in regulating PMN apoptosis. The authors cultured PMN for 0, 2,6 or 22h in the presence of TNFα (pro-apoptotic) or GM-CSF or are left untreated. Fresh, unstimulated PMN showed a high level of expression of Bcl-XL that gradually decreased as the culture proceeded, suggesting that loss of this protective protein may play a role in spontaneous apoptosis. The reduction of Bcl-XL in the presence of TNFα is much stronger when compared to control cells. GM-CSF did not alter the effect of Bcl-XL. Next the investigators examined expression of Bax-α, a proapoptotic member of the BC1-2 family. Results showed that GM-CSF induced a down regulation of Bax-α when compared to control cells, suggesting that the down-regulation of this death promoting is involved in PMN survival mediated by GM-CSF. The authors concluded that GM-CSF seems to promote survival by modulating the Bax-α/ Bcl-XL ratio via down regulation of Bax-a. Furthermore, the authors suggested that inhibition of apoptosis by GM-CSF might be due to a caspase 3 regulation since no further reduction of apoptosis is observed, above that already seen, when PMN are stimulated GM-CSF after inhibition of caspase-3 with its inhibitor Z-DEVD-FMK.

Other members of the BCL-2 family have also been implicated in neutrophil apoptosis. Expression of myeloid cell leukaemia 1 (MCL1), another viability-promoting family member, has been shown to decrease during neutrophil apoptosis but increases in response to GM-CSF and LPS, suggesting a link with PMN survival, (Moulding DA, Quayle JA, Hart CA, Edwards SW, Blood 1998; 92(7): 2495-502). Neutrophils also express mRNA for A1, another BCL2 homologue with anti-apoptotic properties (Chuang PI, Yee E, Karsan A, Winn RK, Harlan JM, Biochem Biophys Res Commun 1998; 249(2): 361-5). The authors demonstrated that agonists that promote cell survival (e.g. LPS and G-CSF) up-regulated the message for this protein. Moreover, neutrophil apoptosis is enhanced in mice that lack A1-a, a subtype of the A1 gene, and LPS- induced inhibition of apoptosis is abolished. However in these mice TNFα induced apoptosis is unchanged, which suggest that A1 is involved in regulating some but not all neutrophil apoptotic pathways (Hamasaki A, Sendo F, Nakayama K, Ishida N, Negishi I, Nakayama Ki, Hatakeyama S, J Exp Med 1998; 188(11):1985-92).

Thus although a few pieces of the apoptotic pathway are in place, key events regulating PMN apoptosis and growth factor induced survival factors are still poorly understood as indeed they are in other cell types.

### Summary of the Invention

The present inventors hypothesised that if a model was designed such that the course of apoptosis following induction and its subsequent inhibition by GM-CSF was functionally characterised both in terms of mechanism and time course, then changes, or patterns of changes, in the 'early' regulatory events could be studied. Studies of changes in expression of those genes involved in these early events, would lead to the identification of potential therapeutic targets. Thus, the present inventors have set out to provide a model system for the GM-CSF mediated inhibition of apoptosis such that those genes involved in the early regulatory events of apoptosis, can be identified and characterised.

The control of apoptosis represents a significant therapeutic target, since many diseases are due to defects in this process. Many physiological factors prevent cell apoptosis. For example cytokines or growth factors inhibit death through apoptosis. There is an acute need to identify the genes that regulate this process. In other words, if one identifies a gene that prevents apoptosis, then this gene/gene product or its function can be blocked by a drug and apoptosis allowed to occur. To-date many of the genes found have certain fundamental flaws e.g. they act late in the process, after the cell has committed to a death programme, or they are ubiquitous, that is they are not restricted to a particular cell type. The ideal targets to control apoptosis act early in the process and are restricted to a particular cell type.

The inventors have discovered that GM-CSF inhibits death through apoptosis by the regulation of 'effector genes' that control the process of apoptosis. A signal acts through a signal transduction cascade and is associated with significant changes, or patterns of changes, in gene expression in the cell. If model discovery assays are configured to target these 'early' regulatory events occurring in the inhibition of apoptosis it is possible to identify the key genes to control apoptosis.

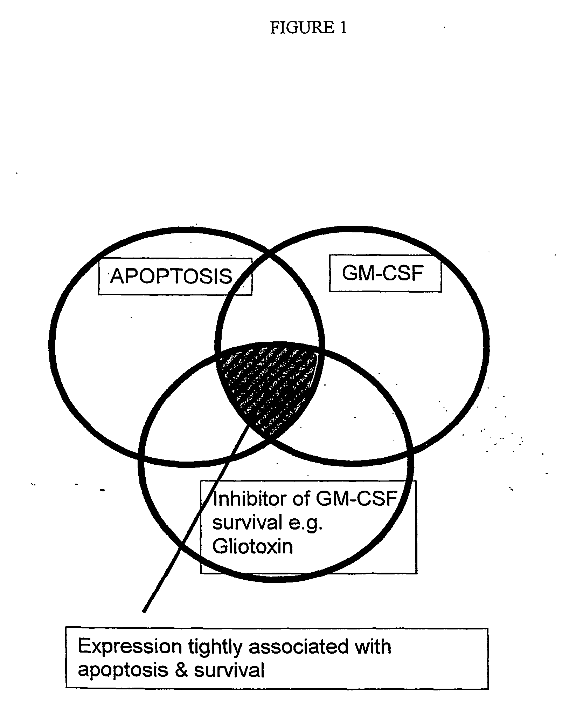

The present inventors have further discovered that if a model is designed such that the inhibition of apoptosis by GM-CSF is itself inhibited by a drug, then changes, or patterns of changes can be targeted by clustering those changes that are common and both increase and/or decrease depending on the treatment. For example, a change that is a 'decrease' following induction of apoptosis is a candidate target gene. Moreover, a change that is additionally an 'increase' following inhibition of apoptosis by GM-CSF has a higher probability of being a target gene because its regulation shows increased correlation with the process. Likewise, a change that is further a 'decrease' following inhibition of GM-CSF inhibitory effect has a yet higher probability of being a target gene because its regulation shows increased correlation with the process. This concept is illustrated by Figure 1, which shows correlation between induction of apoptosis, inhibition of apoptosis by GM-CSF and inhibition of GM-CSF-mediated survival by an inhibitor e.g. gliotoxin. The central shaded region indicates changes associated with all three treatments.

Thus, in a first aspect the present invention provides a method for identifying a gene product which modulates the transition of a cell between a non-apoptopic state and an apoptopic state, comprising the steps of:
(a) exposing the cell to an inhibitor of GM-CSF mediated inhibition of apoptosis; and
(b) exposing the cell to one or more agents which increase tyrosine phosphorylation; and
(c) placing the cell in conditions which permit it to undergo spontaneous apoptosis; and
(d) monitoring the level(s) of expression of the one or more gene products in the cell; and
(e) identifying gene product(s) whose expression has been increased, decreased or modified as a result of performing steps (a) to (d).

In a preferred embodiment of this aspect of the invention, an additional step is introduced of; determining the level(s) of expression of one or more gene product(s) in a cell to establish a reference expression level;

The present inventors have demonstrated that gliotoxin blocks GM-CSF inhibition of apoptosis. This effect appears selective to GM-CSF, in that gliotoxin does not itself increase the rate of neutrophil apoptosis. The effect of Gliotoxin is mediated, at least in part, via NFκB and/or by a mechanism involving NAD(P)H. Accordingly, in a preferred embodiment of this aspect of the invention, gliotoxin is used to inhibit GM-CSF inhibition of apoptosis.

The DNA binding ability of NFκB may be modified by administration of agents known to increase levels of NFκB. Examples of suitable reagents includes the addition of LPS, TNFα, transfection of p65 or p50 components of NFκB. It should be understood that this list is by no means exhaustive. The DNA binding ability of NFkB may be decreased by agents known to inhibit the migration of NFκB into the nucleus, for example,. CAPE (Caffeic acid phenethyl ester), lactacystin, and the transfection of IκB analogues.

In addition, the inhibitory effect of GM-CSF on neutrophil apoptosis, may be blocked by agents known to inhibit the function of NADPH oxidase. Suitable agents include, but are not limited to any one or more of the following: phenyl arsine oxide, diphenylene iodonium. One skilled in the art will be aware of other suitable inhibitors.

The intracellular GM-CSF concentration may be increased by administering exogenous GM-CSF, or by inducing endogenous GM-CSF production in the cell. The latter may be performed by methods known to those skilled in the art, and will be described infra.

Agents suitable to increase tyrosine phosphorylation include phenylarsine oxide (PAO). Tyrosine phosphorylation mimics the effect of GM-CSF. Accordingly, in an alternative embodiment of this aspect of the invention, GM-CSF may be used to substitute for agents which increase tyrosine phosphorylation in step (c) of the method of the present invention..

The intracellular tyrosine phosphorylation concentration may be altered by administering agents which inhibit tyrosine kinase, for example Genestein, or agents which inhibit tyrosine phosphatases, for example Phenylarsine Oxide. Those skilled in the art will be aware of other suitable agents.

It has been found that exposure of the cell to GM-CSF leads to inhibition of apoptosis in the cell at a different rate than occurs under identical conditions but in the absence of GM-CSF. The assay of the invention may be configured to identify gene products that accelerate or retard the induction of apoptosis. In a preferred embodiment of this aspect of the invention, the assay detects gene products that inhibit the induction of apoptosis.

Likewise, it has been found that exposure of the cell to agents which increase the tyrosine phosphorylation leads to inhibition of apoptosis in the cell at a different rate than occurs under identical conditions but in the absence of increased tyrosine phosphorylation. The method of this aspect of the invention may be configured to identify gene products that accelerate or retard the induction of apoptosis.

In addition, it has been found that exposure of the cell to agents which increase the NFκB-DNA binding leads to inhibition of apoptosis in the cell at a different rate than occurs under identical conditions but in the absence of increased NFκB-DNA binding. The assay of the invention may be configured to identify gene products that accelerate or retard the induction of apoptosis. Advantageously, the assay detects gene products that inhibit the induction of apoptosis.

The method of the invention is applicable to the discovery of gene products, and the genes encoding them, which are involved in apoptosis. The gene products may be polypeptides and/or RNAs. "Polypeptide" herein refers to any peptide comprising two or more amino acids, whether comprising a single domain or multiple domains, and includes multi-subunit proteins, which are cellular gene products. RNA gene products include ribozymes, antisense RNA molecules and/or mRNA molecules. Advantageously, the gene products are natural gene products, that is they are encoded by naturally-occurring genes in the cell being investigated and are assembled in the cell using natural components such as amino acids or nucleotides. However, the invention also encompasses screening for gene products encoded by genes that are not endogenous to the cell being investigated. Such genes may be, for example, heterologous genes from other cells or organisms, artificial genes encoding polypeptides comprising domains from different sources or composite RNA molecules, and wholly or partially randomised genes encoding repertoires of polypeptide or nucleic acid gene products.

Levels of gene expression may be determined in any appropriate manner. Preferably, the invention comprises the measurement of protein production by mRNA translation, and is configured to detect increases or decreases in the rate or amount of mRNA translation. The invention may also be configured to detect changes in post-translational processing of polypeptides or post-transcriptional modification of nucleic acids. For example, the invention may be configured to detect the phosphorylation of polypeptides, the cleavage of polypeptides or alternative splicing of RNA, and the like. Levels of expression of gene products such as polypeptides, as well as their post-translational modification, may be detected using proprietary protein assays or techniques such as 2D polyacrylamide gel electrophoresis. Polypeptide or nucleic acid populations may be assessed individually, or together, in order to identify candidate gene products.

Advantageously, expression levels are assessed by measuring gene transcription; preferably carried out by measuring the rate and/or amount of specific mRNA production in the cell. A preferred embodiment of this aspect of the invention involves the use of arrayed oligonucleotide probes capable of hybridising to mRNA populations. Differences in hybridisation patterns of different mRNA populations may be used to identify genes that are differentially expressed in the two populations. The arrayed oligonucleotide probes are advantageously derived from cDNA or EST libraries, and represent genes that are expressed by the cells under investigation.

As used herein, the terms "oligonucleotide" and "polynucleotide" are equivalent, and imply no limitation as to maximum or minimum length.

A reference expression level, once established for a given cell type, may be used for repeated screens, thus facilitating the performance of repeated rounds of screening.

Cells useful in the method of the invention may be from any source, for example from primary cultures, from established cell lines, in organ culture or *in vivo.* Cell lines useful in the invention include fibroblast cell lines, carcinoma cell lines such as neuroblastoma cell lines and cell lines of haematopoietic origin. Preferred are primary cultures of neutrophils or cells having neutrophil characteristics, for example HL-60 cells.

Increases in the NFκB DNA binding can be achieved by treatment of the cells with LPS, TNFα, and p65/p50 transfections.

A number of methods are known in the art for monitoring the onset of apoptosis. These include morphological analysis, MTT assay, Crystal Violet, DNA ladder formation, externalisation of membrane phospholipid phosphatidylserine and caspase activation analysis.

The ability of GM-CSF to inhibit apoptosis is preferably confirmed by monitoring the onset thereof according to one or more of the above methods. Moreover, GM-CSF inhibitors, such as anti-GM-CSF antibodies may be used to further substantiate the role of GM-CSF. Techniques for monitoring the onset of apoptosis and antibody technology will be familiar to those skilled in the art.

The ability of increased tyrosine phosphorylation to inhibit apoptosis is preferably confirmed by monitoring the onset thereof according to one or more of the above methods. Moreover, agents that inhibit tyrosine phosphorylation, such as Genestein may be used to further substantiate the role of increased intracellular tyrosine phosphorylation levels.

Genes regulated in these models following modulation of apoptosis include genes that 1) are 'effector' genes involved in the cells defence mechanisms aimed at preventing apoptosis (anti-apoptotic genes) and thus represent therapeutic targets, 2) make up aspects of the apoptosis and/or GM-CSF signal cascade and thus represent therapeutic targets, 3) initiate the process of apoptosis (pro-apoptotic genes) and thus represent therapeutic targets, and 4) are associated with the processes of apoptosis and defence that will aid in the understanding of key pathways, processes and mechanisms that may subsequently lead to the identification of therapeutic targets.

In a further aspect, the present invention provides the use of gliotoxin to inhibit the GM-CSF mediated inhibition of apoptosis.

In a final aspect, the present invention provides a system for modelling GM-CSF mediated inhibition of apoptosis in a cell comprising the steps of:
(a) the provision of a population of cells;
(b) exposing the cell to an inhibitor of GM-CSF mediated inhibition of apoptosis; and
(c) exposing the cell to an agent which increases tyrosine phosphorylation and/or; exposing the cell to GM-CSF; and
(d) placing the cell in conditions which allow it to undergo spontaneous apoptosis; and
(e) analysing the gene expression of the cell population; and
(f) assessing the onset of apoptosis in said cell population.

### Brief description of the figures

**Figure 1** shows the correlation between the induction of apoptosis, inhibition of apoptosis by GM-CSF and the inhibition of GM-CSF-mediated survival by an inhibitor, for example gliotoxin. The central shaded region indicates changes associated with all three treatments.

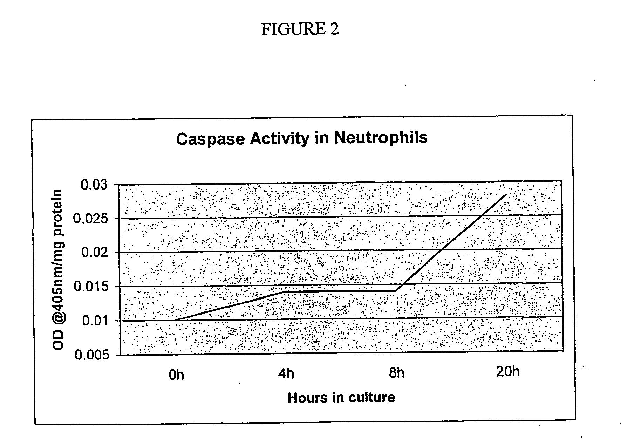

**Figure 2** shows the caspase activity present in the supernatant fraction of human neutrophils which have undergone spontaneous apoptosis. The activity is measured using absorbance at 405nm and normalised per mg of protein. Figure 2 shows the caspase activity during the incubation period. There is a dramatic increase between 8 and 20h of culture.

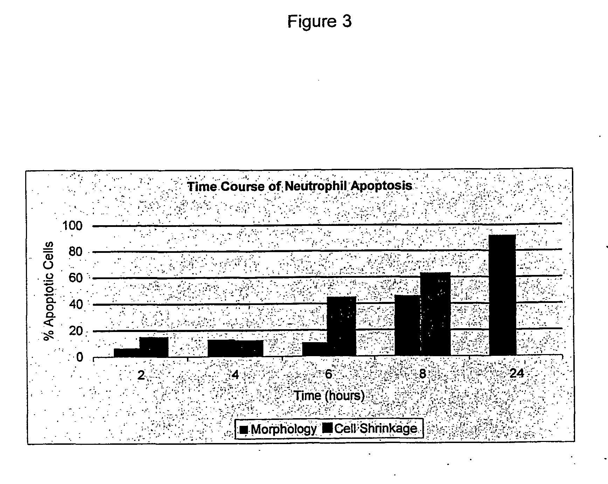

**Figure 3** shows a morphological determination of apoptosis. Cells are removed from culture and centrifuged at 300g for 10 minutes at 4°C. The pellet is kept on ice, washed in ice cold hanks buffer and then re-suspended in cell lysis buffer. Details are given in example 1. Apoptosis as measured by morphology, increased from approximately 6 hours following isolation of the cells. There is a large increase in the number of cells undergoing spontaneous apoptosis between 6-8 hours resulting close to 50% of the cells showing apoptotic morphology at 8 hours.

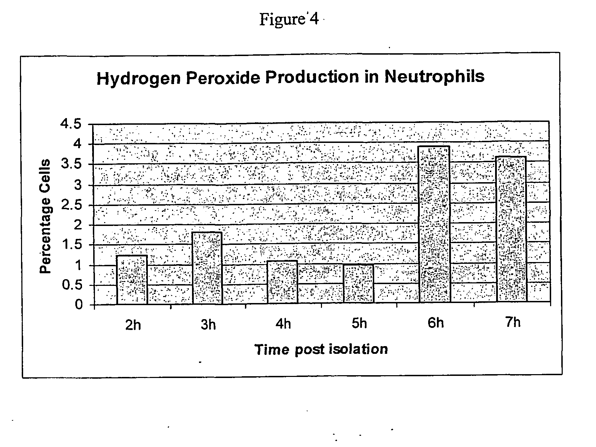

**Figure 4** shows that neutrophil spontaneous apoptosis is accompanied by the endogeneous of ROS, in the form of O₂⁻ and H₂O₂. Experimental details are given in Example 1.

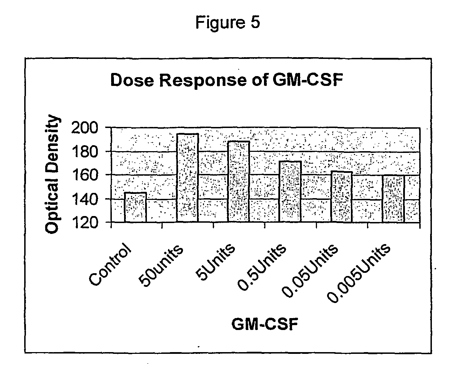

**Figure 5** shows the dose responsiveness of the anti-apoptotic effect of GM-CSF. Optical densities are read at 570 using a plate reader. The results indicate a direct correlation between survival and concentrations of GM-CSF added to the culture medium.

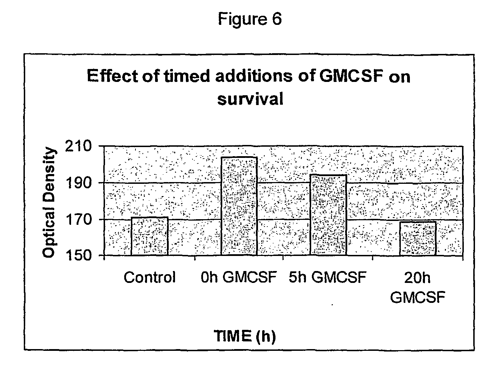

**Figure 6** shows a determination of the temple relationship between GM-CSF addition and survival. A time course of GM-CSF additions to neutrophils is carried out in order to determine up to what stage GM-CSF can induce survival in neutrophils. The method is described in detail in example 1. Optical densities are read at 570 using a plate reader. The results demonstrate that GM-CSF can be added to neutrophils as late as 5 hours post isolation and not result in any discernible loss in the protective effect of this cytokine.The data also indicates that the commitment to die for the vast majority of neutrophils is later than 5 hours post isolation.

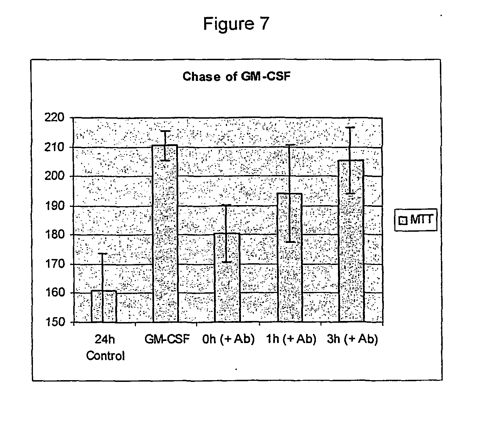

**Figure 7** shows timed additions of anti GM-CSF antibody to neutrophils in order to determine how immediately the anti-apoptotic effects induced by GM-CSF ocurr. The method is described in detail in example 1. Optical densities are read at 570nm using a plate reader. Comparable survival is obtained when addition of anti GM-CSF is delayed for 3 hours following isolation as to when no neutralizing antibody is added. In contrast, when neutralizing antibody is added before the 3 hours, survival is directly linked to the addition time. This indicates that the survival mediated by GM-CSF is not immediate and that cells must be exposed to the cytokine for a defined period of time before survival is conferred.

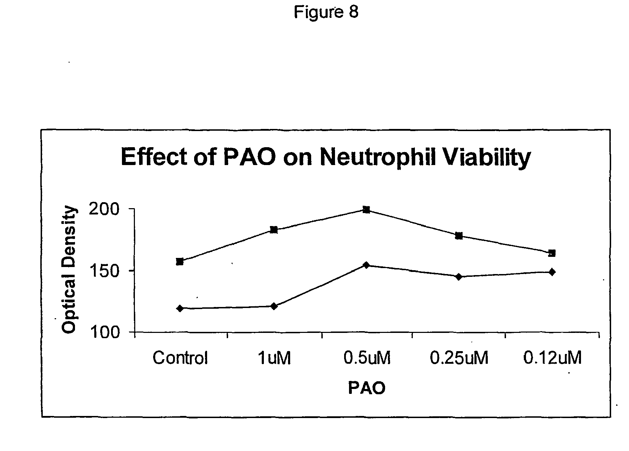

**Figure 8** shows the role of intracellular phosphorylation in delaying the apoptotic process in neutrophils. The addition of low doses of the phosphorylation inhibitor Phenylansine Oxide (PAO) to neutrophils increases the survival of its cells after 24 hours in culture. The method is described in example 2. Optical densities are read at 570 nm using a plate reader. The results show that intracellular tyrosine phoshorylation plays a pivotal role in the protective effects on anti-apoptotic agents described in the study.

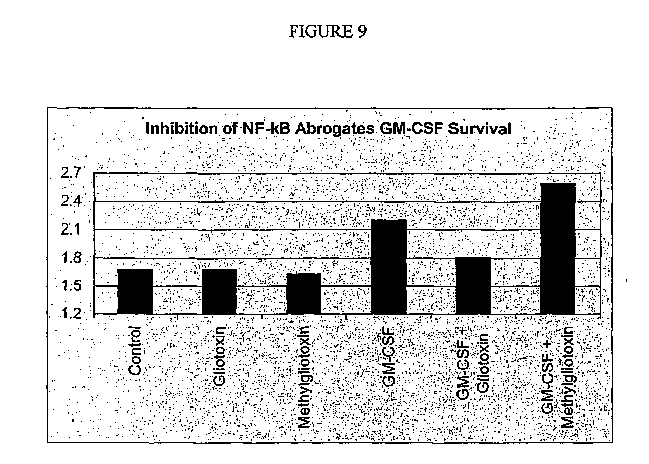

**Figure 9** shows that the fungal metabolite gliotoxin blocks the GM-CSF in the inhibition of neutrophil apoptosis. The method is as described in example 3. Optical densities are read at 570nm using a plate reader. Gliotoxin effectively blocks the GM-CSF mediated inhibition of neutrophil apoptosis. The blocking effect is not seen when the inactive analogue of gliotoxin, methogliotoxin is added with GM-CSF. No increased neutrophil apoptosis is seen with the addition of gliotoxin alone to isolated neutrophils, demonstrating that the effect is specific to, and limited to, a reversal of the protective effects of GM-CSF.

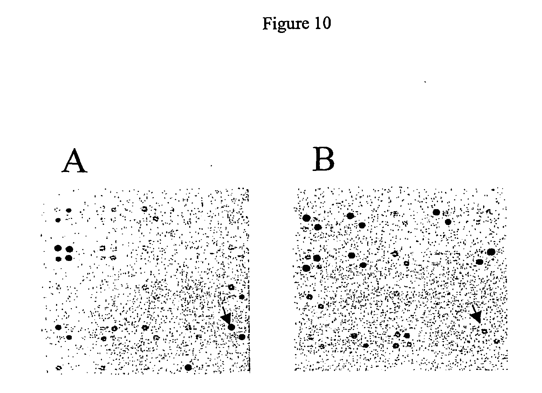

**Figure 10** shows the use of micoarray to confirm the differential expression of SSH clones. A detailed method is described in example 4. Approximately 100 colonies are isolated from each reciprocal SSH cDNA library. cDNA insert sequences are amplified by PCR, and spotted onto microarray filters as described above. Duplicate filters are hybridised radio-labeled cDNA probes generated from the reciprocal RNA material used to generate each SSH library. An analysis of the filters identifies which cDNA clones are differentially expressed.

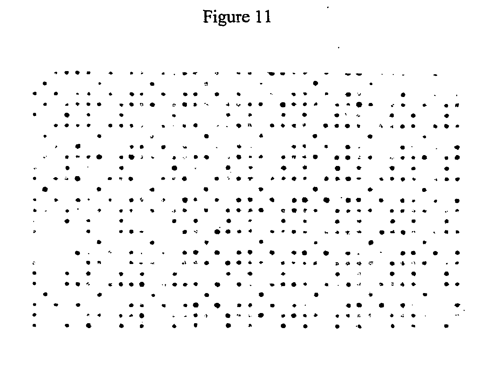

**Figure 11.** Positive clones from the first round of screening described in example 4 and fig 10 were subsequently submitted to GeneScreen for preparation of a micro array. This array is then hybridised against radiolabelled probes of choice to further analyse the expression profiles of the mRNAs corresponding to the isolated DNA sequences.

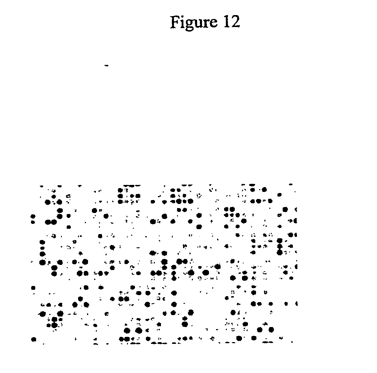

**Figure 12** shows the use of a STORM phosphoimager to quantitively image positive signals across the filter arrays. Hybridised filters are wrapped in plastic wrap and exposed to a low energy phosphoimaging screen. The screen is then placed on a phospho imager and the gel image captured by scanning at a resolution of 50 microns. Full experimental details are given in Example 4.

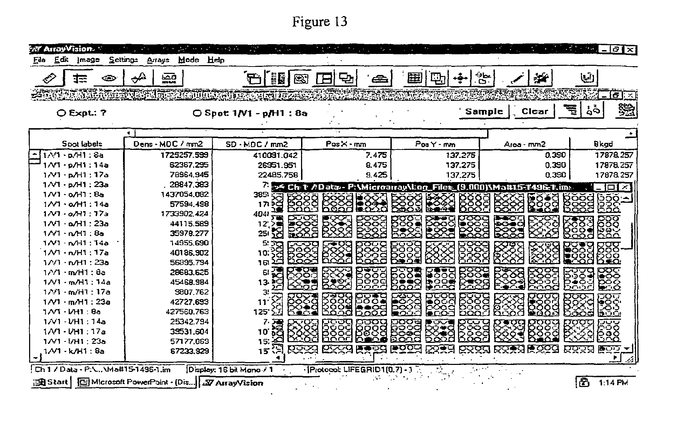

**Figure 13.** The captured image file is then analyzed using software such as ArrayVision, the program contains facilities as spot detection and quantification, and background detection and quantification. The data is then exported to a text file for further analysis.

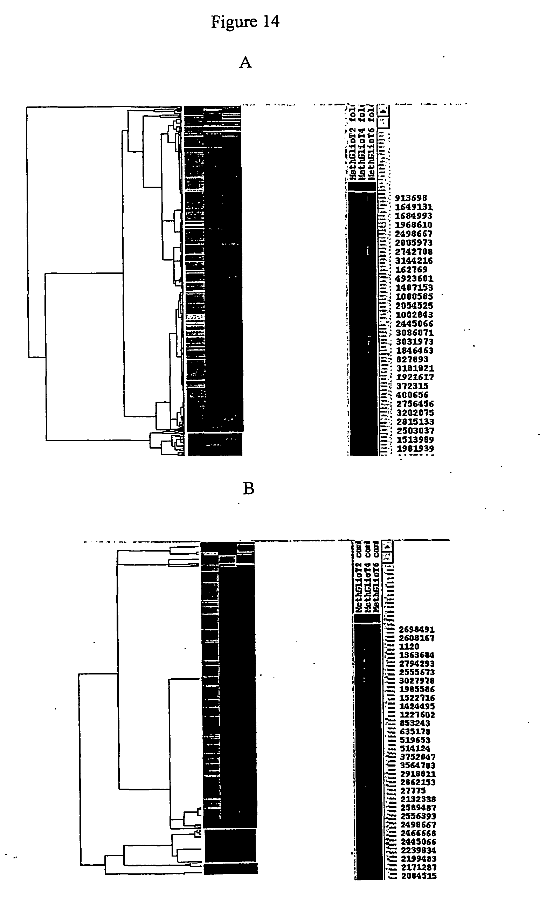

**Figure 14.** Following cluster analysis fold-change data can be difficult to interpret owing to either a very large data set and/or a wide range in fold change values. The interpretation of this data may be simplified using codes or combined codes. The use of combined codes can greatly simplify the Cluster analysis and subsequent visualization.

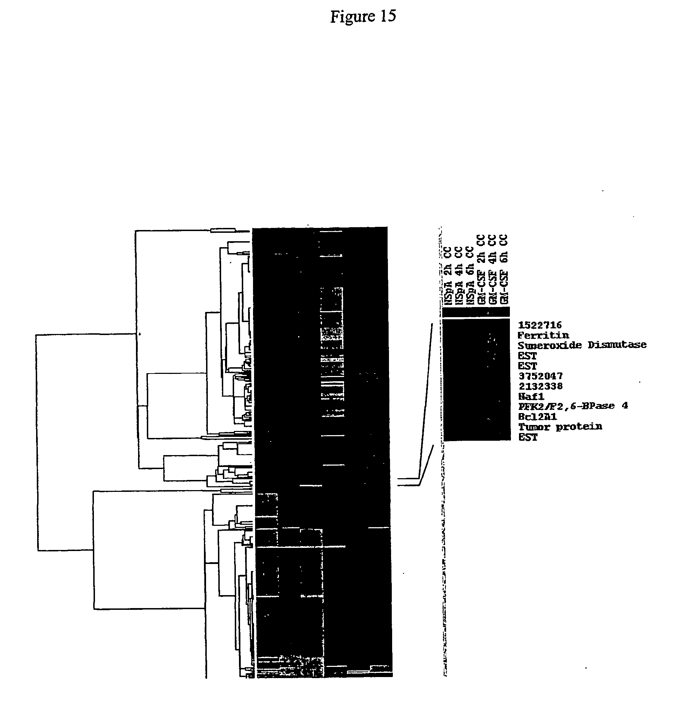

**Figure 15** shows that GM-CSF inhibition of neutrophil apoctosis is associated with significant changes in global mRNA expression. Microarray analysis of gene expression changes associates with GM-CSF inhibition of neutrophil apoptosis was carried out using LifeGrid microarray filters. The details method is described in example 5. A significant number of genes are down regulated following addition of GM-CSF and these also represent candidate target genes.

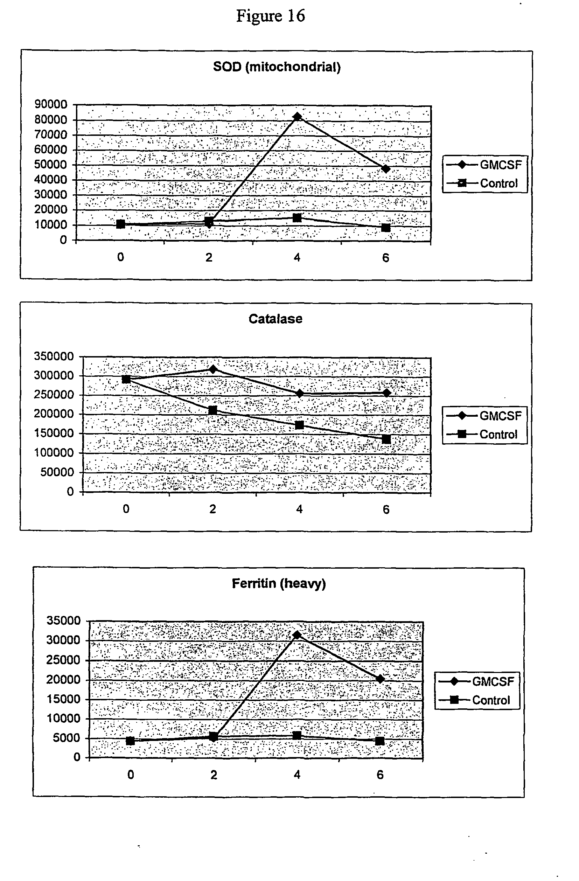

**Figure 16**. A comparison of co-ordinate patterns of gene expression by bio informatic data analysis using the model system of the present invention allows the identification of cell pathways and processes associated with apoptosis and survival. Following cluster analysis a cluster of genes were identified that contained several genes involved in neutrophil survival and whose transcription is unregulated upon GM-CSF treatment. This cluster is termed the survival cluster. Among these genes, one group that could be clearly identified are those genes whose protein products are involved in protecting the cell against an increased oxidative environment.

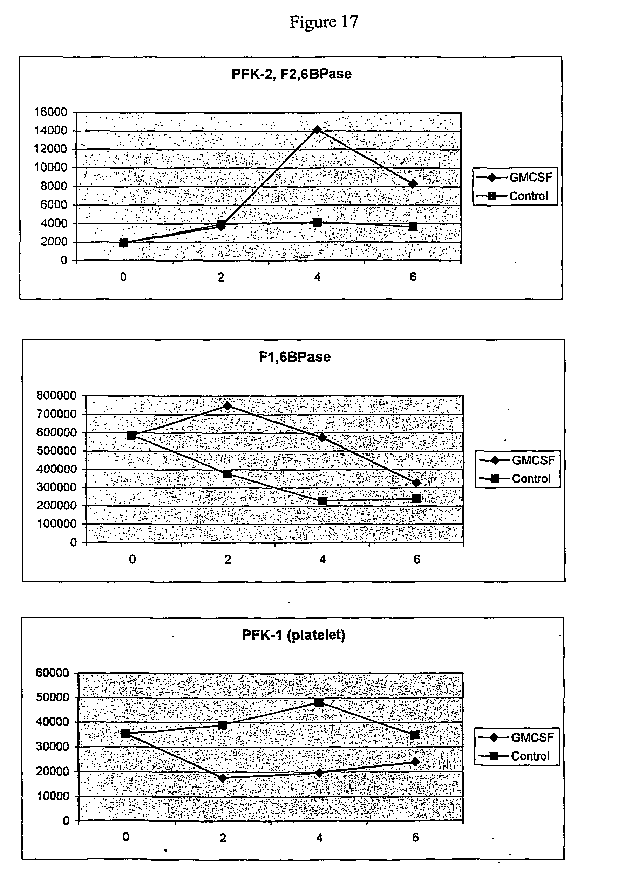

**Figure 17** on further analysis of the survival cluster we identified transcription of the biofunctional enzyme phoshofructokinase-2 (PKF-2)/ fructose 2,6-biphosphatase (FBPase-2) which regulates the cellular concentration of fructose 2,6 bisphosphate and which in turn can regulate the function of phosphofructokinase-1.

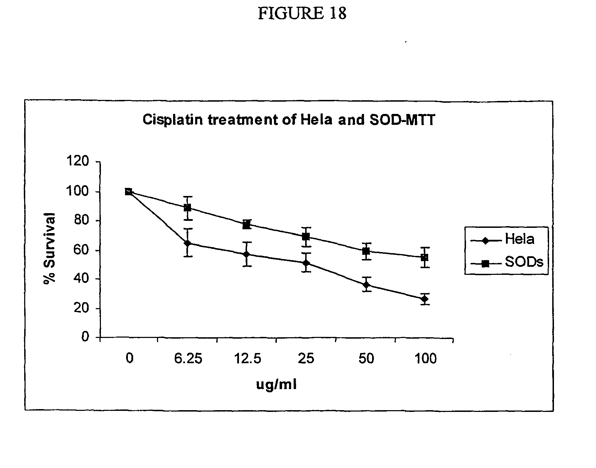

**Figure 18** shows that ectopic expression of superoxide dismutase in Hela cells shows a dose-dependent resistance to apoptosis induced by cisplatin, confirming that superoxide has an anti-apoptotic function.

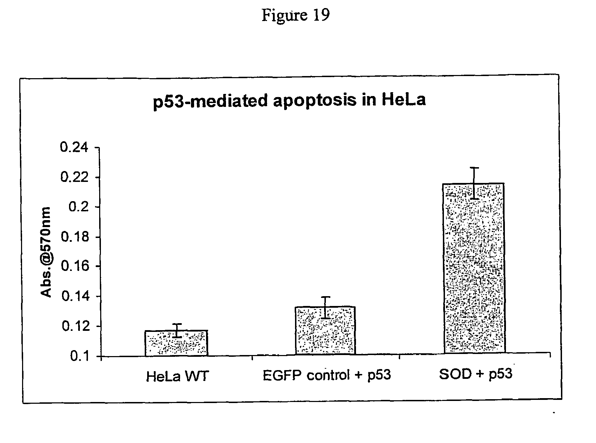

**Figure 19** shows that the ectopic expression of superoxide dismutase in HeLa cells confers resistance to apoptosis induced by expression of the transcription factor p53.

### Brief Description of the Tables

**Table 1** shows a morphological determination of apoptosis. The table shows that although there is a small amount of apoptosis occuring two hours post isolation, there is a large increase in the number of cells undergoing spontaneous apoptosis between 6-8 hours, with close to 50% of the cells showing apoptotic morphology at 8 hours.

**Table 2** shows that an increase in superoxide production, as detected by flow cytometry occurs during the spontaneous apoptosis of neutrophils.

**Table 3** summarizes those genes regulated early in GM-CSF mediated inhibition of neutrophil apoptosis, as identified by Suppression Subtractive hybridisation (SSH). A sample of clones differentially expressed in these libraries are sequenced as described. A number of clones correspond to known genes, whilst many others are unique and do not have any identity with any expressed sequence in the public databases. The sequence identities are presented in table 3.

**Table 4** shows the GM-CSF mediated inhibition of neutrophil apoptosis is associated with significant changes in global mRNA expression. Table 4a. All 17 genes upregulated by GM-CSF at 2 hours are blocked by gliotoxin. Table 4b, of 76 genes upregulated by GM-CSF for 4 hours, 60 are blocked by gliotoxin.Table 4c, another 156 genes upregulated by GM-CSF at 2 hours, 57 are blocked by gliotoxin.

### Detailed description of the invention

The regulation of many genes presently known to be involved in apoptosis, including caspase genes, is presently being investigated as a route to the manipulation of apoptotic processes. Such attempts suffer from a fundamental flaw, in that because many of these genes/proteins become involved in apoptosis after the cell has made a commitment to the apoptotic pathway, regulation of these genes can only serve to delay the onset of apoptosis and not to prevent it. The present invention avoids this fundamental drawback and provides a method by which genes involved in the early stages of apoptosis, whose expression is modulated by GM-CSF levels in the extracellular environment, can be identified and isolated.

The control of apoptosis in neutrophils is useful in the treatment of a number of diseases, including asthma, COPD, posttraumatic acute respiratory distress syndrome (ARDS), systemic lupus erythematosus (SLE), Inflammatory Bowel disease (IBD), end-stage renal disease (uremia), Cardiopulmonary bypass, rheumatoid arthritis, cutaneous allergic (leukocytoclastic) vasculitis (CAV), cystic fibrosis (CF), severe congenital neutropenia (SCN), Endotoxin (ET)-induced liver failure, acute myelogenous leukaemia and neutropenia following radiation and chemotherapeutic treatments for cancer.

The present invention provides a model system for identifying a gene product/s which modulate the transistion of a cell between a non-apoptopic state and an apoptopic state, by exposing the cell to an inhibitor of GM-CSF mediated inhibition of apoptosis; and then exposing the cell to GM-CSF and then identifying gene products whose expression has been modulated as a result of the treatment.

The present invention seeks to overcome the problems encountered by previous attempts aimed at identifying those genes involved in apoptosis. Many previous genomics studies have looked at one parameter eg. Apoptosis. This has resulted in an unwieldy number of candidate genes. Other studies have described cluster analysis across multiple experiments. The model system of the present invention differs in that it permits focused, multiple and opposing events to be studied, and the genes/polypeptides involved in these multiple events to be identified and characterised. For example, Apoptosis vs survival vs the inhibition of survival may be studied.

### General Techniques

Unless defined otherwise, all technical and scientific terms used herein have the same meaning as commonly understood by one of ordinary skill in the art (e.g., in cell culture, molecular genetics, nucleic acid chemistry, hybridisation techniques and biochemistry). Standard techniques are used for molecular, genetic and biochemical methods (see generally, Sambrook *et al*., Molecular Cloning: A Laboratory Manual, 2d ed. (1989) Cold Spring Harbor Laboratory Press, Cold Spring Harbor, N.Y. and Ausubel *et al*., Short Protocols in Molecular Biology (1999) 4^{th} Ed, John Wiley & Sons, Inc. which are incorporated herein by reference), chemical methods, pharmaceutical formulations and delivery and treatment of patients.

### Determination of expression levels

As indicated above, a number of individual gene product types may be screened for in the present invention. These products include polypeptides and nucleic acids. The expression levels assessed may be absolute levels of production of a particular polypeptide or nucleic acid, or the levels of production of a derivative of any polypeptide or nucleic acid. For example, the invention may be configured to measure the level of expression of a particular mRNA splice variant, or the amount present of a phosphorylated derivative of a particular polypeptide.

Where it is desired to monitor the levels of expression of a known gene product, conventional assay techniques may be employed, including nucleic acid hybridisation studies and activity-based protein assays. Kits for the quantitation of nucleic acids and polypeptides are available commercially.

Where the gene product to be monitored is unknown, however, methods are employed which facilitate the identification of the gene product whose expression is to be measured. For example, where the gene product is a nucleic acid, arrays of oligonucleotide probes may be used as a basis for screening populations of mRNA derived from cells.

### Arrays

Gene Arrays of oligonucleotides specific to gene sequences archived in public domain databases, such as GenBank, are available commercially from a number of suppliers (such as Incyte Genomics). Examples of such commercial arrays are in the form of either nucleotides spotted onto a membrane filter (such a nitrocellulose), or a solid support (such as glass). Commercial Gene Arrays are used to profile the patters of gene expression that are associated with the process of apoptosis in neutrophils.

Gene Arrays are additionally constructed in-house, by spotting nucleotide sequences derived from cDNA clones generated from in-house libraries or from cDNA clones purchased commercially. Such arrays allow the expression profiling of proprietary and/or novel nucleotide sequences.

Many of the cDNA sequences or EST (expressed sequence tag) sequences deposited in the public domain databases are derived from a restricted set of tissue types, such as liver, brain and foetal tissue. The cloning of in-house cDNA libraries that are focused to specific cellular events, such as inhibition of apoptosis by GM-CSF offers the possibility to identify, clone and characterise novel genes that are associated with this process. Similarly, the cloning of in-house cDNA libraries that are focused to specific tissue types, such as the neutrophil, offers the possibility to identify, clone and characterise novel genes whose expression is restricted to this cell type. Libraries (cDNA) constructed using a physical subtraction, such as the ClonTech 'Select' SSH (suppression hybridisation) method, allow the selective cloning of genes whose expression is differentially regulated in the process or cell type being studied. Gene Array technology is combined with SSH cDNA libraries to identify false-positives and further focus on truly differentially expressed genes. Clones from each SSH library constructed are picked, cultured and archived as glycerol stocks. The cDNA inserts contained within individual plasmid clone are PCR amplified and spotted onto in-house arrays. Differential expression is confirmed using hybridisation with a radiolabelled probe generated from the mRNA used for each reciprocal subtractions.

Arrays of nucleic acids may be prepared by direct chemical synthesis of nucleic acid molecules. Chemical synthesis involves the synthesis of arrays of nucleic acids on a surface in a manner that places each distinct nucleic acid (e.g., unique nucleic acid sequence) at a discrete, predefined location in the array. The identity of each nucleic acid is determined by its spatial location in the array. These methods may be adapted from those described in U.S. Patent No. 5,143,854; WO90/15070 and WO92/10092; *Fodor et al*. (1991) *Science,* 251: 767; Dower and Fodor (1991) *Ann. Rep. Med. Chem*., 26: 271.

In a preferred aspect of the invention, arrays of nucleic acids may be prepared by gridding of nucleic acid molecules. Oligonucleotides may be advantageously arrayed by robotic picking, since robotic techniques allow the most precise and condensed gridding of nucleic acid molecules; however, any technique, including manual techniques, which is suitable for locating molecules at discrete locations on a support, may be used.

The gridding may be regular, such that each colony is at a given distance from the next, or random. If molecules are spaced randomly, their density can be adjusted to statistically reduce or eliminate the probability of overlapping on the chosen support.

Apparatus for producing nucleic acid microarrays is available commercially, for example from Genetix and Genetic Microsystems. Moreover, pre-prepared arrays of nucleic acid molecules are available commercially, for example from Incyte Genomics Inc. (Human LifeGrid⁽™⁾). Such arrays will comprise expressed sequence tags (ESTs) representative of most or all the genes expressed in a cell or organism, thus providing a platform for the screening of mRNA populations from multiple ROS-treated cells.

Samples for mRNA population analysis may be isolated and purified by any suitable mRNA production method; for example an RNA isolation kit is available from Stratagene.

### 2D PAGE

For the monitoring of unknown polypeptide gene products, separation techniques such as 2 dimensional gel electrophoresis are employed. 2D PAGE typically involves sample preparation, electrophoresis in a first dimension on an immobilised pH gradient, SDS-PAGE electrophoresis in a second dimension, and sample detection. Protocols for 2D PAGE are widely available in the art, for example at http://www.expasy.ch /ch2d/protocols/, the contents of which as of 16.12.1999 are incorporated herein by reference.

Samples for 2D PAGE may be prepared by conventional techniques. In the case of one of the preferred cells for use in the invention, HL60 (promyelocytic leukemia cells), a monolayer culture of a human promyelocytic leukemia cell line is grown in a suitable medium, such as RPMI 1640 containing 10% foetal calf serum (FCS), and treated with GM-CSF as necessary. The suspension is transferred into a tube and the cells are centrifuged at 1000 g for 5 minutes. Supernatant is discarded and the cells are washed with RPMI 1640 without FCS. After centrifugation and removal of RPMI 1640, 0.8 x 10⁶ cells are mixed and solubilised with 60 µl of a solution containing urea (8 M), CHAPS (4% w/v), Tris (40 mM), DTE (65 mM) and a trace of bromophenol blue. The whole final diluted HL60 sample is loaded on the first dimensional separation.

The method of the present invention advantageously employs a step of establishing a reference expression level for the gene products being investigated. This can be carried out before addition of GM-CSF to the cells, and serve as a standard for one or more subsequent assays; or it may be an integral part of every assay. For example mRNA or polypeptide populations from GM-CSF treated and untreated cells may be assessed simultaneously on a nucleic acid array or by 2D PAGE, and changes in expression patterns identified by direct comparison.

Analysis of 2D PAGE results, using appropriate software where necessary, reveals Polypeptides of interest may be isolated, sequenced and used to identify genes encoding them.

### General Applications of the Invention

The model systems or discovery assays described herein provides the means to characterise the molecular mechanisms of apoptosis. In particular, these molecular mechanisms include the GM-CSF mediated inhibition of apoptosis. In addition, the strategy provides the means to identify, characterise, and clone molecules, including oligonucleotides and polypeptides, associated, both causally and consequentially, to the GM-CSF mediated inhibition of apoptosis.

We have shown that gliotoxin inhibits the GM-CSF mediated inhibition of apoptosis. By using an inhibitor of GM-CSF in a model of the GM-CSF mediated inhibition of apoptosis, the genes and gene products involved in multiple events may be identified and characterised.

In a preferred configuration of the present invention, cells such as primary human neutrophils are isolated and purified from the peripheral blood of individuals. Upon culture in a serum-containing cell culture medium these neutrophils undergo 'spontaneous apoptosis' (Haslett, Clinical Science 83, pp639-648, 1992). Apoptosis as measured by morphology is apparent and increases from approximately 6 to 8 hours following isolation of the cells. The onset of apoptosis is further characterised using additional markers for apoptosis, such as caspase activation and cell shrinkage. These assays serve to identify the earliest measurable onset of the cells commitment to apoptosis. Intracellular events which drive this commitment of the cell to apoptosis occur before and around this earliest measurement of the commitment.

In a further preferred configuration of the present invention, cells such as HL60 or HeLa, are cultured and treated with external agents that induce apoptosis through the endogenous production of ROS, such as UV-irradiation, cytokines such as TNFα, or chemotherapeutic agents such as cisplatin. Upon treatment of cells with UV-irradiation, TNFα, or cisplatin, apoptosis is induced; this apoptosis has been shown to be mediated by an endogenous production of ROS (Miyajima A, Nakashima J, Yoshioka K, Tachibana M, Tazaki H, Murai M (1997) Role of reactive oxygen species in cis-dichlorodiammineplatinum-induced cytotoxicity on bladder cancer cells. Br J Cancer 76 (2) pp206-10; Chan, W-H, Yu, J-S (2000) Inhibition of UV Irradiation-induced oxidative stress and apoptotic biochemical changes in human epidermal carcinoma A431 cells by genestein. J. Cell Biochem. 78 pp73-84; Sidoti-de Fraisse C, Rincheval V, Risler Y, Mignotte B, Vayssiere JL (1998) TNF-alpha activates at least two apoptotic signalling cascades. Oncogene (13): pp1639-51). Apoptosis as measured by morphology is apparent as determined by DNA fragmentation. The onset of apoptosis is characterised using markers for apoptosis. These assays serve to identify the earliest measurable onset of the cells commitment to apoptosis. Intracellular events which drive this commitment of the cell to apoptosis occur before and around this earliest measurement of the commitment.

The characterisation of the apoptosis process and GM-CSF involvement in this model system permit the key early intracellular events which control the GM-CSF mediated inhibition of apoptosis, the molecular signalling pathways mediating or associated with GM-CSF mediated inhibition of apoptosis.

We have shown that gliotoxin inhibits the GM-CSF mediated inhibition of apoptosis and that the effect is mediated at least in part via NFkB and/or NAD(P)H. In addition, the model system has been used to show that the protective effect of GM-CSF is mediated, at least in part, via tyrosine phosphorylation.

Global gene expression patterns allows a detailed characterisation of the mechanisms underlying and causal to the apoptotic process. Indeed we show GM-CSF mediated inhibition of apoptosis involves an increase in intracellular tyrosine phoshorylation in neutrophils. In addition, we show that genes regulated early in GM-CSF mediated inhibition of neutrophil apoptosis include superoxide dismutase (Mn-SOD), and Hypoxia Inducible factor (HIFalpha). A number of other genes were identified whose gene products are involved in apoptosis and survival including those whose protein products are involved in protecting the cell against an increased oxidative environment. These include catalase and ferritin as well as superoxide dismutase. Thus, we conclude that GM-CSF protects the cell by reducing the harmful effects of reactive oxygen species.

In addition, phosphofructokinase-2 (PFK-2)/fructose 2,6-biphosphatase (FBP ase-2) transcription is increased. PFK-2 can regulate the cellular concentration of fructose 2,6 biphosphate, and in turn the activity of phosphofructokinase-1. GM-CSF also causes up-regulation of fructose 1,6 biphosphatase and down regulation of phosphofructokinase 1. These enzymes are components of the pentose-phosphate pathway. From these studies it could be concluded that GM-CSF increases the formation of glucose 6 phosphate which can be utilised in reducing NADPH via the pentose phosphate pathway.

Analysis of global gene expression patterns, using for example 'cluster analysis' to group genes with similar temporal patterns of expression, allows a detailed characterisation of signalling pathways and cellular processes associated with treatments, as detailed above. Indeed we show that this analysis, of gene expression using our model system, identifies genes that are 'known' to be co-regulated and co-expressed, and which belong to the same signalling pathways or cellular process. We also show that many genes (the expression patterns of which are grouped, or clustered, with those genes that have know function) identified and cloned have little or no know known biological function, or which have no existing sequence homology to previously cloned genes. These genes may represent the most attractive therapeutic targets.

The invention provides the means to allow the identification and isolation of genes or polypeptides that may represent valuable and attractive therapeutic targets for the control of apoptosis in a range of conditions and diseases.

### Example 1.

Establishment and characterisation of a 'Model Cell-System / Discovery Assay' to study the molecular mechanisms of cellular response to GM-CSF mediated inhibition of apoptosis in primary human neutrophils.

This example describes the establishment of a model cell system in primary human neutrophils. The isolation and culture of primary human neutrophils is described. Neutrophils are allowed to undergo spontaneous apoptosis in culture. This spontaneous apoptosis is accompanied and characterised by the endogenous production of ROS. This spontaneous apoptosis is inhibited by the addition of GM-CSF. This 'Model Cell System' forms the basis of a novel 'Discovery Assay' that is then used for the characterisation, identification and functional validation of nucleotide (genes, mRNAs) or polypeptide (proteins, peptides) sequences associated with and potentially responsible for the molecular mechanisms of apoptosis, including; 1) the cells attempt to combat apoptosis in the cell, 2) the cellular signaling pathways, 3) the initiation of the apoptosis process, and 4) are associated with the processes of apoptosis and defence that will aid in the understanding of key pathways, processes and mechanisms.

We demonstrate the utility of this model system to 'discover' early regulated genes associated with GM-CSF inhibition of apoptosis, by the discovery of genes that have 'known' involvement in cell survival. Similarly we demonstrate the identification of genes regulated 'early' in GM-CSF inhibition of apoptosis that have no known biological function i.e. ESTs from both commercial filters and from our own libraries of differentially expressed genes isolated by Suppression Subtractive Hybridisation (SSH). By association with known genes, as described, these novel genes themselves are identified as candidate apoptosis genes that may similarly function in 1) the cells attempt to combat apoptosis in the cell, 2) the cellular signaling pathways, 3) the initiation of the apoptosis process, and 4) are associated with the processes of apoptosis and defence that will aid in the understanding of key pathways, processes and mechanisms.

### Isolation and culture of primary human neutrophils

Whole blood (20-50 ml) is taken from normal healthy volunteers by venepuncture. Coagulation is prevented by the use of sodium citrate. A 6% dextran (mol wt 509,000; Sigma) saline solution is added in 1:4 ratio to whole blood and the erythrocytes allowed to sediment for 45 minutes at 22°C. The buffy coat is then under-layered with 5 ml Ficoll-Paque (Pharmacia LKB Biotechnology) and centrifuged (300g, 30 min) to pellet granulocytes and erythrocytes (Boyum, 1968). The pellet is resuspended in 1 ml cell culture tested water (Sigma) for 40 sec., followed by the addition of 14ml Hanks buffer (Sigma) and centrifuged (300g, 10 min.). This lysis step is repeated to ensure removal of all erythrocytes. The remaining pellet is resuspended in RPMI 1640 supplemented with 10% foetal calf serum (Sigma), L-glutamine (2mM), penicillin (100 U/ml; Sigma), streptomycin (100 µg/ml; Sigma) and amphotericin B (2.5 µg/ml; Sigma). Cell number and viability is checked using trypan blue exclusion (Boyum, (1968) *Scand J Clin Lab Invest Suppl;* 97:77-89).

Isolated neutrophils are maintained at a density of 2 x10⁶/ ml in RPMI 1640 supplemented with 10% foetal calf serum (Sigma). Further additions to the medium included L-glutamine (2mM), penicillin (100 U/ml), streptomycin (100 µg/ml) and amphotericin B (2.5 µg/ml) (Sigma). Cells are incubated at 37°C in a humidified CO₂ (5%) incubator.

### Cellular/biochemical characterisation of apoptosis

A range of assays are established and used to measure the magnitude and temporal induction of apoptosis. The earliest biochemical measurement of the apoptosis phenotype by these assays is considered to be the point beyond which the cells are 'Committed' to the process of apoptosis. In addition, these measurements determine the reproducibility of induction of apoptosis in the model systems. Furthermore, these measurements determine the cellular mechanisms of apoptosis in these systems (such as whether apoptosis is caspase-dependent or caspase-independent).

Primary human neutrophils are isolated and purified from the peripheral blood of normal healthy individuals. Upon culture in a serum-containing cell culture medium these neutrophils undergo 'spontaneous apoptosis' (Haslett, Clinical Science 83, pp639-648, 1992).

Our data indicate the earliest detection of neutrophil spontaneous apoptosis, and thus a time of 'commitment to die' at 6 to 8 hours post-isolation.

### Caspase activation assay

Caspase activity (Caspase-3) is measured using a commercial kit (CaspACE™ Assay System, Promega). The methodology is essentially as described by the manufacturer. Cells are removed from culture and centrifuged (300g/ 10 min) at 4°C. The pellet is kept on ice, ished in ice-cold Hanks buffer and then resuspended in Cell Lysis Buffer at 10⁸/ ml. Cells are lysed by freeze-thaw once, incubated on ice for 15 min, followed by centrifugation (15,000g /20 min) at 4°C. The caspase 3 activity present in the supernatant fraction is measured using the absorbance at 405nm and normalised per mg of protein in the supernatant. Figure 2 represents the increase in caspase activity during the incubation period. While caspase activity is observed at the earlier time points, there is a dramatic increase between 8 and 20h of culture, which is in agreement with our morphological data.

### Morphological Determination of Apoptosis

A cell aliquot (100µl) is removed from culture and using an IEC Centra-7 centrifuge equipped with a Cytobucket ™ adapter, a monolayer of cells is concentrated onto standard microscopic slides. Preparations are allowed to air dry prior to fixing in Rapi-Diff (Diagnostic Developments, UK) solution A (reactive ingredient 100% methanol). Slides are air dried prior to immersion in solution B containing; eosin Y (0.1% w/v), formaldehyde (0.1% w/v), sodium phosphate dibasic (0.4% w/v) and potassium phosphate monobasic (0.5% w/v). Excess stain is drained from the slide prior to immersion in solution C, containing methylene blue (0.4%w/v), Azure A (0.04% w/v), sodium phosphate dibasic (0.4% w/v), potassium phosphate monobasic (0.05% w/v) and potassium phosphate monobasic (0.4% w/v), to counterstain the cytoplasm. Excess dye is rinsed; the slides air-dried and mounted in DPX aqueous mountant (BDH Laboratory Supplies, U.K.). Morphological examination is then carried out by light microscopy for the presence of apoptotic cells as determined by the loss of membrane asymmetry and condensation of cytoplasm and nuclei (Cotter and Martin, 1996).

Apoptosis as measured by morphology is apparent and increased from approximately 6 hours following isolation of the cells. Our results demonstrate that although there is a small amount of apoptosis occurring at 2h post isolation, there is a large increase in the number of cells undergoing spontaneous apoptosis between 6 -8h resulting in close to 50% of the cells showing apoptotic morphology at 8 hours (See Table 1 and Figure 3).

### Cell Shrinkage Determination of Apoptosis

Onset of apoptosis as determined by cell shrinkage is determined by flow cytometry. Isolated cells are set up in culture at a concentration of 2x10⁶/ml. At the indicated time points a sample is removed and the forward and side scatter parameters of the cells are measured by flow cytometry using a Becton Dickenson FACScan equipped with CellQuest software. Cell shrinkage is detected by a reduction in forward scatter parameters. Determination of apoptosis inhibition is calculated by the percentage of cells with reduced forward scatter parameters, in untreated cultures, minus percentage of cells with reduced forward scatter parameters in treated cultures.

Our results for spontaneous neutrophil apoptosis demonstrate that although there is a small amount of apoptosis occurring at 2h and 4h post isolation, there is a large increase in the number of cells undergoing spontaneous apoptosis from 6h with close to 50% of the cells showing apoptotic morphology at 6 hours (See Figure 3).

### Neutrophil spontaneous apoptosis is accompanied by the endogenous production of ROS, in the form of O₂⁻ and H₂O₂

Human neutrophils are specialised for the phagocytosis and lysis of bacteria. This lysis is accompanied by an enzyme complex called NADPH oxidase (Babior, B.M. (1978)). Oxygen dependent microbial killing by phagocytes. New England Journal of Medicine, 298(12) pp659-668. NADPH oxidase is assembled at the membrane of the phagosome and generates ROS, in the form of superoxide (O₂⁻). This endogenous production of ROS contributes to the bacterial lysis. NADPH oxidase activity is present in normal peripheral blood neutrophils and is further increased by cellular activation. Consequently, neutrophils have the capacity to generate significant amounts of endogenous ROS. As with other cell types, ROS in the form of superoxide may also be produced by mitochondria during oxidative respiration.

Chronic Granulomatous Disease (CGD) is characterised by a molecular defect and loss of activity of the neutrophil NADPH Oxidase. Notably, neutrophils isolated from patients with CGD have a significantly delayed spontaneous apoptosis Kasahara Y, Iwai K, Yachie A., Ohta, K., Konno A., Seki H., Miyawaki, T and Taniguchi N. (1997) Involvement of reactive oxygen intermediates in spontaneous and CD95(Fas/Apo-1)- mediated apoptosis of neutrophils. Blood 89 (5) pp1748-1753.

Increased H₂O₂ occurs upon neutrophil activation, with. e.g. PMA, and this is associated with increased rate of apoptosis (Lundqvist-Gustafsson H, and Bengtsson T. (1999) Activation of the granule pool of the NADPH oxidase accelerates apoptosis in human neutrophils. J. Leuk. Biol. 65: pp196-204).

Our results demonstrate that we can detect an increase in the superoxide production, as detected by flow cytometry (See Table 2). The greatest number of cells producing superoxide anions peaked 3-4 hours post isolation, before returning to background levels.

Our results also demonstrate that this peak in O₂⁻ production precedes an increase in intracellular H₂O₂ production (See Figure 4). This temporal pattern is consistent with the H₂O₂ production resulting from the action of superoxide dismutase, an enzyme present in neutrophils and which catalyses the conversion of O₂⁻ to H₂O_{2.}

### Dose responsiveness of the anti-apoptotic effect of GM-CSF.

Primary human neutrophils are isolated and purified from peripheral blood of normal healthy individuals. Neutrophils are resuspended in serum containing culture medium together with various amounts of GM-CSF at a density of 2x10⁶/ml, with 100µl plated into a 96 well plate and cultured for 18h at 37°C. After this time 10µl of MTT (5mg/ml) is added to the cultures and incubated for a further 4h at 37°C before solubilisation of the purple coloured formazan with acidic isopropanol. Optical densities are read at 570nm using a plate reader. Our results demonstrate a direct correlation between survival and concentrations of GM-CSF added to the culture medium (Figure 5).

### Determination of the temporal relationship between GM-CSF addition and survival

A time course of GM-CSF additions is carried out to neutrophils in order to determine up to what stage GM-CSF can induce survival in neutrophils. Primary human neutrophils are isolated and purified from peripheral blood of normal healthy individuals. Neutrophils are resuspended in serum containing culture medium at a concentration of 2x10⁶/ml, with 100µl/well plated into a 96 well plate and culture at 37°C commenced. At the indicated time points GM-CSF (50U/ml) is added to the neutrophils and culture continued until 20h post initiation of culture. After this time, 10µl of MTT (5mg/ml) is added to the cultures and incubated for a further 4h at 37°C before solubilisation of the purple coloured formazan with acidic isopropanol. Optical densities are read at 570nm using a plate reader. Our results demonstrate that GM-CSF can be added to neutrophils as late as 5 hours post isolation and not get any discernible loss in the protective effect of this cytokine. However, if administration of the cytokine is delayed for 20h post isolation, the protective effect is zero in 24h cultures. This data indicates that the commitment to die for the vast majority of neutrophils is later than 5 hours post isolation (See Figure 6). This result is in agreement with our other findings that morphological characteristics of apoptosis occurs between 6 and 8 hours.

### Determination of GM-CSF "on time" sufficient for apoptosis delay

We performed timed additions of anti-GM-CSF antibody to neutrophils in order to determine how immediate the anti-apoptotic effects induced by GM-CSF occur. Primary human neutrophils are isolated and purified from peripheral blood of normal healthy individuals. Neutrophils are resuspended in serum containing culture medium containing 5 U/ml of GM-CSF at a concentration of 2x10⁶/ml, with 100µl/well plated into a 96 well plate and culture at 37°C commenced. At the indicated time points additions 10µg/ml anti-GM-CSF are made to the neutrophils and cultured for 18h at 37°C. After this time, 10µl of MTT (5mg/ml) are added to the cultures and incubated for a further 4h at 37°C before solubilisation of the purple coloured formazan with acidic isopropanol. Optical densities are read at 570nm using a plate reader. Comparable survival is obtained when addition of anti- GM-CSF is delayed for 3 hours following isolation as to when no neutralising antibody is added (See Figure 7). In contrast, when neutralising antibody is added before 3 hours, survival is directly linked to the addition time. This indicates that the survival mediated by GM-CSF is not immediate and that cells must be exposed to the cytokine for a defined period of time ("on time") before survival is conferred.

### Example 2

### Intracellular Tyrosine Phosphorylation is required for GM-CSF inhibition of neutrophil apoptosis.

This example characterises the role that intracellular phosphorylation plays in delaying the apoptotic process of neutrophils. As can be seen in Fig 8, the addition of low doses of the phosphatase inhibitor Phenylarsine Oxide (PAO) to neutrophil increase the survival of the cells after 24h in culture. Therefore it can be concluded that intracellular tyrosine phosphorylation plays a pivotal role in the protective affects on anti-apoptotic agents described in this study.

Primary human neutrophils are isolated and purified from peripheral blood of normal healthy individuals. Neutrophils are resuspended in serum containing culture medium at a concentration of 2x10⁶/ml and 100µl aliquots plated into a 96 well plate. The protein phosphatase inhibitor Phenylansine Oxide (PAO) is added to select wells at time 0h, at various concentrations (0.1 - 1µM) and culture at 37°C commenced. After the indicated time, 10µl of MTT (5mg/ml) are added to the cultures and incubated for a further 4h at 37°C before solubilisation of the purple coloured formazan with acidic isopropanol. Optical densities are read at 570nm using a plate reader.

### Example 3

### Fungal metabolite Gliotoxin blocks GM-CSF inhibition of neutrophil apoptosis.

This example describes the identification of an inhibitor for the GM-CSF mediated inhibition of neutrophil apoptosis. The use of this inhibitor allows us to focus in on the specific biochemical events mediating the GM-CSF survival events. In turn one is able to remove some of the noise associated GM-CSF treatment.

Primary human neutrophils are isolated and purified from peripheral blood of normal healthy individuals. Neutrophils are resuspended in serum containing culture medium containing 5 U/ml of GM-CSF at a concentration of 2x10⁶/ml. Also added to the culture mix is either 0.1µg/ml of the fungal metabolite Gliotoxin (a specific inhibitor of NFκB) or its inactive analogue bis -Dethio -bis (Methylthio) Gliotoxin, with 100µl/well plated into a 96 well plate and culture at 37°C commenced. After the indicated time, 10µl of MTT (5mg/ml) are added to the cultures and incubated for a further 4h at 37°C before solubilisation of the purple coloured formazan with acidic isopropanol. Optical densities are read at 570nm using a plate reader.

Figure 9 demonstrates that gliotoxin effectively blocks the GM-CSF inhibition of neutrophil apoptosis. This blocking effect is not seen when the inactive analogue of gliotoxin, methylgliotoxin is added with GM-CSF. No increased neutrophil apoptosis is seen with the addition of gliotoxin alone to isolated neutrophils demonstrating that the effect is specific to and limited to a reversal of the protective effects of GM-CSF.

Gliotoxin has been shown to inhibit the transcription factor NKκB (Ward et al. 1999, J. Biol. Chem. 274. 4309-4318).. In turn Yoshida et al have identified an alternative mode of action of gliotoxin. These investigators demonstrated that gliotoxin inhibited NADPH oxidase and consequently prevented the onset of superoxide generation by human neutrophils in response to phorbol myristate. (Yoshida et al Biochem Biophys Res Commun 2000, 268(3) 716-23).

By comparing GM-CSF treated, but specifically inhibited for NFκB (and/or NADPH oxidase) with GM-CSF treated alone, allows us to identify specific genes that fit the criteria of a) transcribed in the presence of GM-CSF b) and whose transcription is mediated via a specific transcriptional factor (and/or signal transduction pathway). Our data demonstrates that NFκB binding to DNA (and/or NADPH oxidase activity) is a prerequisite for GM-CSF mediated survival (Fig 9).

### Example 4

### Characterisation and discovery of oligo/polynucleotides 'Genomics' associated with GM-CSF inhibition of neutrophil apoptosis.

This example describes the characterization, cloning and analysis of oligonucleotide/polynucleotide sequences whose expression changes are associated with GM-CSF inhibition of neutrophil apoptosis.

In one example, Suppression Subtractive Hybridisation is used to identify and clone cDNA sequences derived from differentially expressed genes, associated with neutrophil apoptosis, GM-CSF inhibition of neutrophil apoptosis, and the inhibition of this effect using the fungal metabolite Gliotoxin. Such differential gene expression is associated with a measurable apoptotic phenotype.

In another example, commercial microarrays are used to measure global gene expression associated with GM-CSF inhibition of neutrophil apoptosis, and the inhibition of this effect using the fungal metabolite Gliotoxin.. Analysis of such microarray results identifies genes whose expression pattern changes (either up-regulation or down-regulation) in an association with a measurable apoptotic phenotype.

### Suppression Subtractive Hybridisation (SSH) cloning of differentially expressed genes

This example describes the process of Suppression Subtractive Hybridisation (SSH). SSH is andopen' differential cloning system. Unlike microarray, which requires the analysis of known gene sequences, SSH has the potential to clone all mRNAs that are differentially expressed between a control and test population. We also describe substantial modification to the commercial SSH procedure that enhances its performance.

### Total RNA isolation

Primary or cultured cells are split into control and treatment groups and the treatment group is then challenged with apoptotic stimuli or inhibitors as appropriate. Total RNA is then prepared from both groups using acid phenol/guanidine isothiocyanate extraction (RNAzol B; Biogenesis), or RNeasy RNA preparation kits (Qiagen). Any contaminating genomic DNA is removed by DNase treatment (DNase I, Gibco-BRL). In this example, RNA is prepared from neutrophil cells following treatment with GM-CSF (50units/ml), Gliotoxin (10µM) or MethylGliotoxin (10µM). RNA is also prepared from neutrophils that have not been exposed to drug (i.e. as an untreated control). RNA is prepared from these cells using two sequential extractions with RNAzol B.

### Extraction of mRNA

Total RNA prepared as described above is used to prepare mRNA, using the Oligotex mRNA purification kit (Qiagen), or a similar system (Clontech or Stratagene). Briefly, mRNA is purified by passing the total RNA over an oligo-dT column. The oligo-dT may be attached to cellulose or glass beads or biotin, and the column may be either spin or gravity format. The bound mRNA is subsequently ished and eluted, ready for use in subtractive hybridisation.

### Preparation of SMART cDNA

When the quantity of total RNA is in limiting amounts, SMART cDNA amplification can be used to prepare template for the SSH reaction. SMART cDNA synthesis can start with either total or poly-A⁺ RNA and depends upon a feature of the reverse transcriptase enzyme which causes the addition of unmatched deoxycytidines to the 3'-end of the cDNA. An oligonucleotide which has an oligo(dG) sequence at its 3'end pairs with the deoxycytidine stretch, creating an extended template. The reverse transcriptase can then switch strands and continue replicating to the end of the oligonucleotide. The resulting cDNA contains a primer site at each end, which can be used for PCR. The application of limited rounds of amplification then conserves the relative abundance of each cDNA, while significantly increasing the amount of material available for subsequent manipulation.

In this example, SMART cDNA amplification kits are obtained from Clontech. SMART cDNA is prepared using 1µg of total RNA from each time point. The reverse transcribed material is amplified to 17 cycles (to maintain a linear amplification), following which the cDNA is size fractionated over a Nucleospin chromatography column. SSH is performed on mRNA purified from treatment or control cells essentially as described (Diatchenko *et al* 1996), using a PCR-select cDNA subtraction kit (Clontech, K1804). Briefly, cDNA is synthesised according to the SMART protocol for two treatment groups (driver and tester respectively). The resulting cDNA is digested with a restriction enzyme generating a blunt end product (in this example *RsaI*). The tester cDNA is divided into two subsets and distinct adaptor molecules are ligated to each of the cDNA pools. These two samples of tester cDNA are then separately combined with driver cDNA in a solution containing 50mM HEPES, pH 8.3; 0.5M NaCl; 0.02mM EDTA, pH 8.0. Following denaturation, (1.5min, 98°C), the tester and driver cDNAs are allowed to anneal for 10hrs at 68°C. After this first hybridisation, the two samples are combined and a fresh portion of heat denatured driver cDNA is added. The samples are allowed to anneal for a further 10 hours at 68°C. The hybridised cDNA is then diluted in a solution containing 20mM HEPES, pH 8.3; 50mM NaCl; 0.2mM EDTA) and heated at 72°C for 7 minutes, prior to PCR amplification. PCR is performed under standard conditions using the Advantage cDNA PCR kit (Clontech). Only cDNAs that have the correct primer combination, the differentially expressed cDNAs, will amplify exponentially. Reciprocal subtractions are prepared for each pair of samples, which thereby allows identification of both up and down-regulated genes.

### Construction of SSH cDNA libraries

PCR products from the subtractive hybridisation are inserted into a TA cloning vector, in this example the pAdvanTAge cloning kit (Clontech). This library of differentially expressed cDNAs is then transformed to *E. coli* and the transformants selected for plasmid DNA prep and sequence analysis.

### Plasmid miniprep

This example describes the extraction of double stranded plasmid DNA from cDNA clones.

Individual colonies (E. coli DH5α or TOP10F') are picked into 96-deep well culture blocks containing 2.3 ml LB + ampicillin. The culture blocks are shaken at 300 rpm 37°C for 24 h. Plasmid DNA is isolated using MultiScreen-FB and MultiScreen-NA 96-well plates (Millipore). The methodology is as described by the manufacturer.

### DNA sequencing

This example describes the di-deoxy sequencing of cloned cDNA inserts within the vector pT-AdvanTAge (Clontech).

Plasmid miniprep DNA (100 ng to 5 µg) is sent to MWG Biotech for contract sequencing. Sequencing reactions are primed using one of the following universal primer sequences:
M 13 (-24) Reverse Primer: 5' aac agc tat gac cat g 3'
M13 (-48) Reverse Primer: 5' agc gga taa caa ttt cac aca gga 3'
M13 (-20) Forward Primer: 5' gta aaa cga cgg cca gt 3'
M13 (-40) Forward Primer: 5' gtt ttc cca gtc acg ac 3'
T3 Primer: 5' aat taa ccc tca cta aag gg 3'
T7 Primer: 5' gta ata cga ctc act ata ggg c 3'

### Use of microarray to confirm differential expression of SSH clones

The SSH procedure for the cloning of differentially expressed gene products also generates a portion of artifactual cDNAs 'false positives' which are not differentially expressed. This example describes our modifications to the commercial SSH, by the use of microarray to confirm the identity of truly differentially expressed clones prior to the laborious task of sequencing.

Approximately 1000 colonies are isolated from each reciprocal SSH cDNA library. cDNA insert sequences are amplified by PCR and spotted onto microarray filters as described above. Duplicate filters are hybridised with radio-labeled cDNA probes generated from the reciprocal RNA material used to generate each SSH library (i.e. if an SSH library is made from a subtraction between diseased vs. normal cells, then one filter is hybridised with probe synthesised from RNA isolated from diseased cells and the other from normal cells) i.e. 'first round of screening'. An analysis of the filters identifies which cDNA clones are differentially expressed (See Figure 10).

Positive clones from the first round of screening are subsequently submitted to GeneScreen (Dorset, UK) for preparation of a micro array. This array is then hybridised against radiolabelled probes of choice to further analyse the expression profiles of the mRNAs corresponding to the isolated DNA sequences (See Figure 11).

These arrays may be additionally analysed using the ArrayVision software as described above. The output from this program may be normalised and further analysed using the Cluster and Treeview software, as described above.

### Measurement of global gene expression by 'Microarray'

This example describes the process of microarraying (in the context of both a commercial filter based microarray, or commercial spotted filter microarrays (as described above)) and its use to profile gene expression of thousands of genes simultaneously. The microarray process can be separated into three parts: the filter, the hybridisation of radiolabelled cDNA probes, and the detection and quantitation of the microarray results.

### The microarray filter

This example describes the use of the Human LifeGrid™microarray filters obtained from Incyte Genomics (USA). These filters contain cDNA probes representing approximately 8,400 human mRNAs. This example also describes the use of filter microarrays spotted commercially by GeneScreen (Dorset, UK).

### Synthesis of labelled probes

This example describes the synthesis of a radiolabelled cDNA from total cellular mRNA. The labeled cDNA is used to 'probe' DNA fragments, which have been immobilised on to a filter membrane, by complementary hybridisation.

Methodology is as described by manufacturer, for Human LifeGrid™ arrays. Essentially, total cellular RNA (1 µg to 20 µg) or polyA+ mRNA (100 ng to 5 µg) is incubated with an oligo (dT) primer. Primed RNA is reverse transcribed to first stand cDNA in a reaction containing M-MLV reverse transcriptase (RT; alternatively Superscript II is used (Life Sciences)), RT buffer, dNTPs and [α-³³P] dCTP (2000-4000 Ci/mmol) at 42°C for 1 to 5hours. Unincorporated nucleotides are removed using spin-columns and the labeled probe stored at -80°C until required.

Labeled probes may also be generated from cDNA, genomic DNA or PCR products. In each case a random primed labeling procedure can be used, for example the Ready-Prime Labeling kit (APBiotech), applied as per manufacturers instructions.

### Hybridisation of filter based microarrays

This example describes the complementary hybridisation of radiolabelled cDNA probe to DNA fragments immobilised onto a membrane (typically a nylon or nitrocellulase filter).

Methodology is as described by manufacturer, for Human LifeGrid™ arrays. Essentially, membrane filters are pre-hybridised in hybridisation buffer (5 to 20 ml) at 42°C for 2 to 16 h using a hybridisation oven (Hybaid). Following pre-hybridisation, the labeled cDNA probe is added to fresh hybridisation buffer (5 to 20 ml) and hybridised at 42°C for 14 to 16 h. Following hybridisation, the hybridisation mix is removed and the filters ished with 2 x SSC buffer at RT for 5 min., twice with 2 x SSC, 1% SDS buffer at 68°C for 30 min. and twice with 0.6 x SSC, 1% SDS buffer at 68°C for 30 min.

### Quantitative imaging and analysis of microarray filters

This example describes the use of a STORM Phosphoimager to quantitatively image positive signals across the filter arrays. Hybridised filters are wrapped in plastic wrap (Saran) and exposed to a Low-Energy Phosphoimaging screen (Molecular Dynamics). The screen is then placed on the phosphoimager and the gel image captured by scanning at a resolution of 50 microns (See Figure 12).

The captured image file is then analysed using software such as Array Vision (Imaging Research Inc.; See Figure 13). In this example we implement analysis with ArrayVision v5.1. This program contains facilities for spot detection and quantification, and background detection and quantification. This data is then exported to a text file for further analysis. A variety of data fields are exported from the ArrayVision analysis, including; Spot Label, Position, Density, Background, and particularly, Background subtracted density (sDens) and signal/noise ratio (S/N). In this example; the exported text file is up-loaded to an SQL-7.0 database, to populate a table containing array data from all experiments. As the data is imported to the database, a Normalisation factor is calculated and the sDENs values modified accordingly. This Normalised data is stored in a newly created column within the table. The Normalisation factor facilitates accurate comparison between datasets. A number of different calculations may be used. A normalization factor may be derived from Linear Regression calculated by reference to housekeeping genes. Alternatively, the Global Mean is calculated as the average of the sDens values across all of the arrays to be compared and a normalisation factor is then derived by division of the overall spot density with the Global Mean value. Spot density values (individual sDens) are then corrected by multiplying across all values with the normalisation factor. In a similar approach a Global Geometric Mean normalization factor may be calculated and used to adjust the dataset. The data from multiple hybridisation experiments can then be stored in a suitable format, for example in an Access or SQL 7.0 database.

Comparison between arrays generates an output file containing the gene identifier and the fold-change in expression relative to the reference dataset. Fold change, (*Tx vs Ty*), is calculated by dividing the normalised spot density values of *Tx* with *Ty.* In this example, multiple time-course experiments are prepared and fold-change values calculated with reference to the T0 time point.

The fold change data derived from comparison of multiple hybridisation experiments can be analysed using a variety of approaches, including hierarchical clustering, (supervised or unsupervised), k-means clustering or self-organising maps. Software enabling these analyses includes the Cluster and Treeview software (M.Eisen, Stanford Uni, USA), J-Express (European Bioinformatics Institute), GeneMaths (Applied Maths, Belgium) or GeneSpring (Silicon Genetics, USA). In this example hierarchical clustering is implemented using the GeneMaths software. Trees are generated using the UPGMA algorithm with distance calculated using the Pearson similarity metric. Alternatively Euclidean distance metrics are used.

### Simplification of Fold-change data

Following cluster analysis, fold-change data can be difficult to interpret owing to either a very large dataset and/or a wide range in fold change values. The visualization and interpretation of these datasets may be simplified using codes or combined codes.

In this example, each unique gene is represented by at least two identical cDNAs on the array. The fold change value is calculated as described, then for each spot, a value above 5-fold change is accorded a code of 2, a fold-change value of less then 5 but greater then 2 is accorded a code of 1 and a fold-change value of less then 2 is accorded a code value of 0. A combined code is then derived by adding the code values for each identical cDNA on the array. The use of combined codes can greatly simplify the Cluster analysis and the subsequent visualisation (See Figure 14).

### Genes regulated 'early' in GM-CSF inhibition of neutrophil apoptosis are identified by Suppression Subtractive Hybridisation.

This example describes the use of SSH, with modifications discussed, to identify and clone genes that are regulated 'early' in the process of GM-CSF inhibition of neutrophil apoptosis. Many of these DNA sequences are novel, i.e. they are not recognised by BLAST analysis to public gene sequence databases.

Following subtractions between 2 and 4 hour time points of GM-CSF treatment, with and without the presence of fungal metabolite Gliotoxin, a preliminary screening procedure is applied to arrays of clones generated from the subtracted libraries, (as described). Approximately 1000 clones are screened from each of the four subtracted libraries, yielding 700 differentially expressed clones.

The subtracted cDNA prepared by SSH may be used to probe the LifeGrid Arrays. Comparison of the data from this hybridization with that derived from hybridization with unsubtracted probe provides an indication of
1. the efficiency of the SSH subtraction
2. the gene representation within the subtracted cDNA
3. the overlap between SSH derived probes and probes prepared directly from cellular RNA

Using this approach we demonstrate differential expression of Superoxide Dismutase (Mn-SOD; as is previously identified using LifeGrid microarray filters), Hypoxia Inducible factor (HIFα) and a number of other genes in both the SSH cDNA and the unsubtracted total RNA probes.

A sample of clones differentially expressed in these libraries are sequenced as described. A number of clones correspond to well-characterised genes, while many others are unique and do not have any identity ('Novel Clone) with any expressed sequence in the public databases (GenBank NR and dbEST). The sequence identities are presented in Table 3.

### EXAMPLE 5: GM-CSF inhibition of neutrophil apoptosis is associated with significant changes in global mRNA expression.

This example describes microarray analysis of gene expression changes associated with GM-CSF inhibition of neutrophil apoptosis, using LifeGrid microarray filters. In addition, GM-CSF inhibition of neutrophil apoptosis is blocked by the fungal metabolite Gliotoxin.

In excess of 2,000 genes are differentially expressed. A significant number of genes are up-regulated following addition of GM-CSF (17 genes are increased 2-fold of more 2h following addition of GM-CSF, 76 are increased by 4h and 156 by 6h). These up-regulated genes may represent potential survival factor genes which block the apoptosis in neutrophils. Many of these genes that are up-regulated by GM-CSF are blocked by the fungal inhibitor gliotoxin. These genes are further implicated in the survival effects of GM-CSF and may be regulated via NFκB transcription factor or by signal transduction via NADPH oxidase. All 17 genes up-regulated by GM-CSF at 2h are blocked by Gliotoxin (see Table 4 A). Of 76 genes up-regulated by GM-CSF at 4h, 60 are blocked by Gliotoxin (see Table 4 B). Of 156 genes up-regulated by GM-CSF at 2h, 57 are blocked by Gliotoxin (see Table 4 C).

A significant number of genes are down-regulated following addition of GM-CSF and these also represent candidate target genes (see Figure 15).

### Example 6: Cluster-analysis: Comparison of coordinate patterns of gene expression, by bioinformatic data analysis, using this model system, allows the identification of cell pathways and processes associated with apoptosis and survival.

Primary human neutrophils are isolated and purified from peripheral blood of normal healthy individuals. Neutrophils are resuspended in serum containing culture medium together with GM-CSF (50U) at a concentration of 2x10⁶/ml, and cultured for 0h (control), 2h, 4h and 6h at 37°C. Additionally the fungal metabolite Gliotoxin is included with the GM-CSF treatments to block the GM-CSF mediated inhibition of apoptosis. After these times, total cellular RNA is extracted and this used as the starting material for analysis of gene expression changes using the Incyte "LifeGrid" microarray. Following cluster analysis, a cluster of genes are identified that contained several genes involved in neutrophil survival and whose transcription is up - regulated upon GM-CSF treatment. This cluster, which included Bcl2-A1 (a 'known' antiapoptotic neutrophil BCL2 family member) is termed the 'survival' cluster (See Figure 15). Among these genes, one group that could be clearly identified are those genes whose protein products are involved in protecting the cell against an increased oxidative environment (Fig 16). Thus GM-CSF increased expression of mitochondrial superoxide dismutase (dismutases superoxide to hydrogen peroxide), catalase (converts H₂O₂ to H₂O and O₂) and ferritin (which sequesters iron thus limiting the Fenton reaction and preventing production of hyper-reactive hydroxyl radical) above control levels and this coincided with the ability of GM-CSF to prolong survival in neutrophils. Since all these gene products are attenuators of the oxidative cellular response it is indicative that GM-CSF protects the cell by reducing the harmful effects of reactive oxygen species.

On further analysis of this survival cluster, we identified increased transcription of the bifunctional enzyme phosphofructokinase-2 (PFK-2)/fructose 2,6 -bisphosphatase (FBPase-2) which regulates the cellular concentration of fructose 2,6- bisphosphate and which in turn can regulate the function of phosphofructokinase-1 (See Figure 17). The ability of a cell to be able to reduce its oxidative environment is contingent on the cells ability to produce NADPH. This is particularly important in tissues that carry out the biosynthesis of fatty acids such as the liver and mammary tissue and other cells that are subjected to oxidative stress (Voehringer D.W., Hirschberg D.L., Xiao J., Lu Q., Roederer M., Lock, C.B. Herzenberg L.A., Steinman L., Herzenberg, L.A. (2000) PNAS, 97(6) pp2680-2685). Consequently, genes, which are represented on the array and whose products are known to be important in the pentose phosphate pathway are examined.

GM-CSF upregulates fructose 1,6 bisphosphatase (which functions to convert fructose 1,6 bisphosphate to fructose 6 phosphate which can then reversibly convert to Glucose-6-phosphate, the primary substrate for the pentose phosphate pathway. Simultaneously with up regulation of fructose 1,6 bisphosphatase, GM-CSF down regulated the transcription of phosphofructokinase 1 (whose role is to convert fructose 6 phosphate to fructose 1,6 bisphosphate), again ensuring that the pentose phosphate pathway is favoured above glycolysis. Therefore it is concluded that GM-CSF increases the formation of glucose 6 phosphate which can then be utilised in generation of reducing NADPH via the pentose phosphate pathway.

This survival cluster also contains a number of EST with as yet unknown function, these represent potentially novel target genes (see Figure 15).

### Example 7: In vitro cell based assays to confirm pro- and/or anti-apoptotic role for candidate genes identified by gene expression analysis.

This example describes the establishment and use of a number of cell-based in vitro assays to measure pro- or anti-apoptotic activity by the ectopic overexpression of full-length cDNA coding sequence in a mammalian cell line; HeLa. For this example Mn superoxide dismutase is cloned and confirmed to have anti-apoptotic activity. Superoxide dismutase is identified by both microarray analysis and by SSH analysis of gene expression, and is representative of genes found in survival cluster by this model system.

### Obtaining full-length cDNA sequence.

In this example full length human SOD mRNA sequence is identified from the GenBank Acc# X07834. Full length cDNA coding sequence is amplified by RT-PCR from neutrophil RNA using standard techniques, and cloned into the expression vector pCR3.1-Uni (Invitrogen).

Alternatively, full-length clones are isolated from poly A+ RNA using Smart™ RACE Technology (Clontech) according to the manufacturers protocol. Briefly, double stranded cDNA is generated using the Smart II oligonucleotide and the CDS Primer (both supplied in the kit). Specific PCR products are then generated using the PCR primer (supplied in the kit) and gene specific primers (sense and antisense primers generated from the DNA sequence to be extended). To increase specificity, nested sense and antisense primers are used in secondary PCR amplification. PCR products are then ligated into plasmids, such as the TA cloning system (Invitrogen), transformed into competent cells and expanded. Plasmids are purified using mini-prep isolation system (Qiagen) and plasmids are submitted to MWG for sequencing. Specific 5' and 3' sequences are identified using sense and antisense gene specific primers. Products are to be sequenced approximately 10 bases 5' of the initiation codon for 5' PCR products and 10 bases 3' of stop codons for 3' PCR products. Using sequence data, 5' and 3' primers are made and full-length cDNA is amplified.

### Transfection of HeLa cells to generate stable cell lines.

HeLa cells are seeded (2 x 10⁶) in a T75 tissue culture flask and incubated overnight at 37°C. 36µg plasmid DNA (containing candidate gene cDNA) are transfected overnight using Calfos (as described by manufacturer; Clontech). The Calfos reagent and DNA is removed, the cells ished with PBS and incubated in fresh medium overnight. The following day G418 antibiotic (500µg/ml) is added and the cells incubated for one week, where after they are expanded for use in cell based validation assays.

### Determination of functional impact of ectopic expression of candidate gene on ciplatin-induced apoptosis.

This example describes an assay to measure sensitivity or resistance to cisplatin-induced apoptosis in HeLa cells. Cell lines are generated by transfection, which stably express a candidate gene cDNA, in this example Mn superoxide dismutase. The cells are challenged with a cytotoxic agent, Cisplatin that induces apoptosis in HeLa in a dose dependent fashion.

Stable cell lines are seeded into wells of a 96-well microtitre plate at a concentration of 5000 cells per well. 4 wells of each cell line for each drug concentration are set up and plates incubated overnight. Cisplatin (0-200µg/ml) is added to each well and incubated for 24h, following which cell viability is measured by MTT.

Figure 18 shows that ectopic expression of superoxide dismutase in HeLa cells confers a dose dependent resistance to apoptosis induced by cisplatin, confirming that superoxide has anti-apoptotic function.

### Determination of functional impact of ectopic expression of candidate gene on p53-induced apoptosis.

This example describes an assay to measure sensitivity or resistance to p53-induced apoptosis in HeLa cells. Cell lines are generated by transfection, which stably express a candidate gene cDNA, in this example Mn superoxide dismutase. EGFP-expressing cells are compared as a control. The cells are challenged by transfection and ectopic expression of a full-length cDNA for human p53 transcription factor that induces apoptosis in HeLa.

Stable cell lines are seeded into wells of a 24-well microtitre plate at a concentration of 1.5 x 10⁶ cells per well, and incubated overnight. A plasmid containing full length human p53 coding sequence (Acc# X54156) cloned in pCR3.1 expressing hygromycin antibiotic resistance is transfected into cells using Calfos reagents (according to manufacturers instructions, Invitrogen). Post-transfection cells are washed free of Calfos reagent and allowed to recover overnight and then selected for antibiotic resistance by the addition of 500 µg/ml hygromycin. One week following antibiotic selection cells are washed twice to remove cell debris and stained with 1% crystal violet (in ethanol). Stain is released by extraction with 33% acetic acid and cell viability quantitated spectrophotametrically @570nM.

**Figure 19** shows that ectopic expression of superoxide dismutase in HeLa cells confers resistance to apoptosis by expression of the transcription factor p53.

Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry, molecular biology and biotechnology or related fields are intended to be within the scope of the following claims.

**Table 1**

| | 2h | 4h | 6h | 8h | 20h | 24h |
|---|---|---|---|---|---|---|
| Sample 1 | 6 | 11 | 9 | 44 | 91 | 83 |
| Sample 2 | 7 | 15 | 12 | 48 | - | 93 |

**Table 2.**

| | 1 h | 2h | 3h | 4h | 5h | 6h | 7h |
|---|---|---|---|---|---|---|---|
| Time | | | | | | | |
| Sample 1 | - | 7.61 | 10.77 | 25.85 | 12.38 | 8.34 | 6.75 |
| Sample 2 | 4.35 | 14.72 | 17.32 | 9.86 | 10.17 | 7.46 | 6.99 |

**Table 3**

| Clone ID | Annotation |
|---|---|
| 104 F8 | Novel Clone |
| 106 B7 | NADH-ubiquinone oxoreductase chain 4 |
| 107 F5 | NADH-ubiquinone oxoreductase chain 4 |
| 110 G6 | Plasma glutamate carboxypeptidase (PGCP) |
| 105 D12 | Novel Clone |
| 105 E11 | Novel Clone |
| 105 F2 | Novel Clone |
| 111 A7 | Cytochrome B |
| 111 F12 | ATP Synthase A chain; F0 subunit 6 |
| 111 H12 | Tomoregulin |
| 113 A4 | Hypoxia inducible factor 1 alpha |
| 106 B4 | Novel Clone |
| 107 E3 | Novel Clone |
| 109 C2 | Novel Clone |
| 112 A4 | IL8 |
| 112 B4 | Novel Clone |
| 113 F1 | IL8 |
| 114 F7 | IL8 |
| 105 D1 | Novel Clone |
| 105 F8 | Novel Clone |

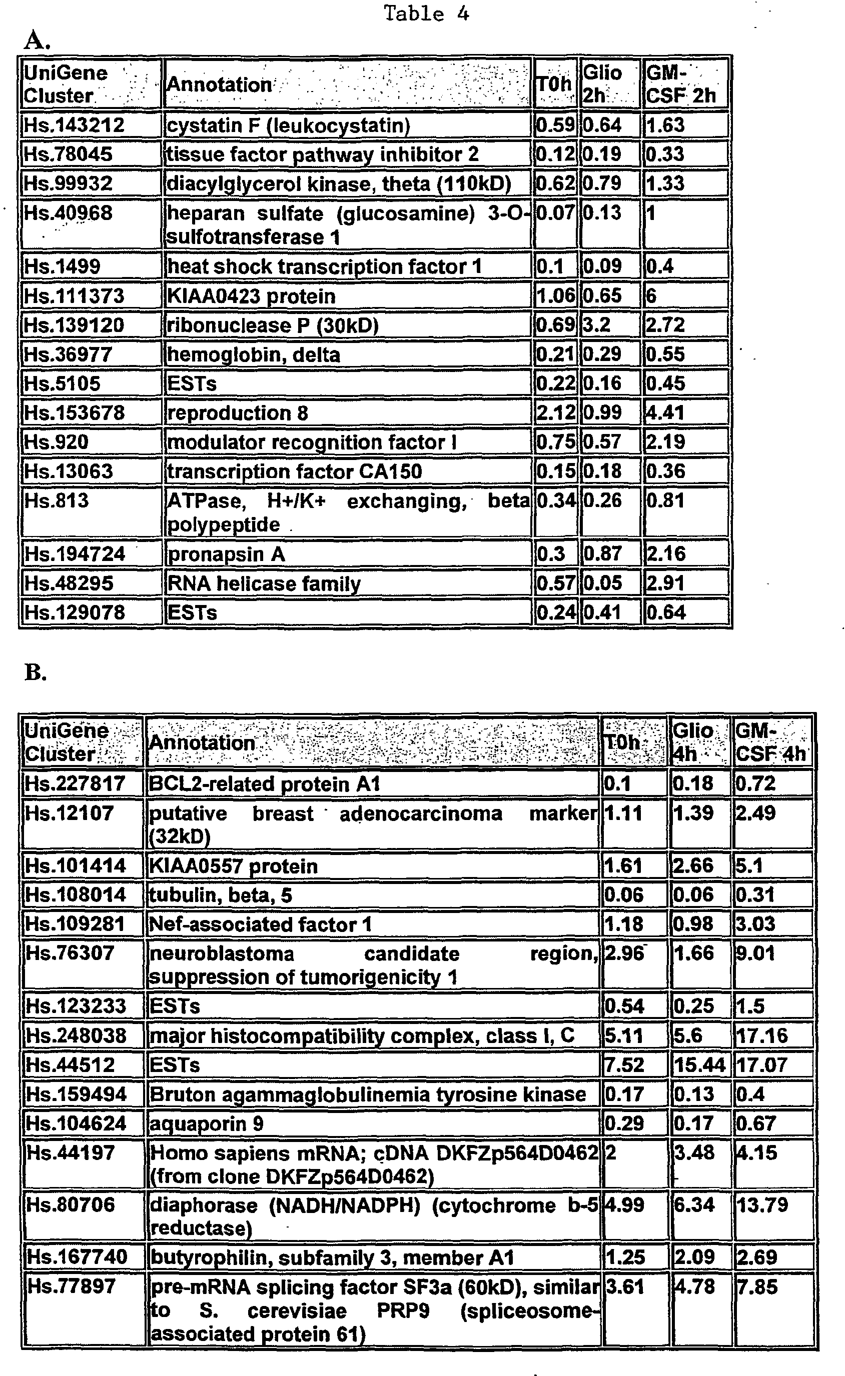

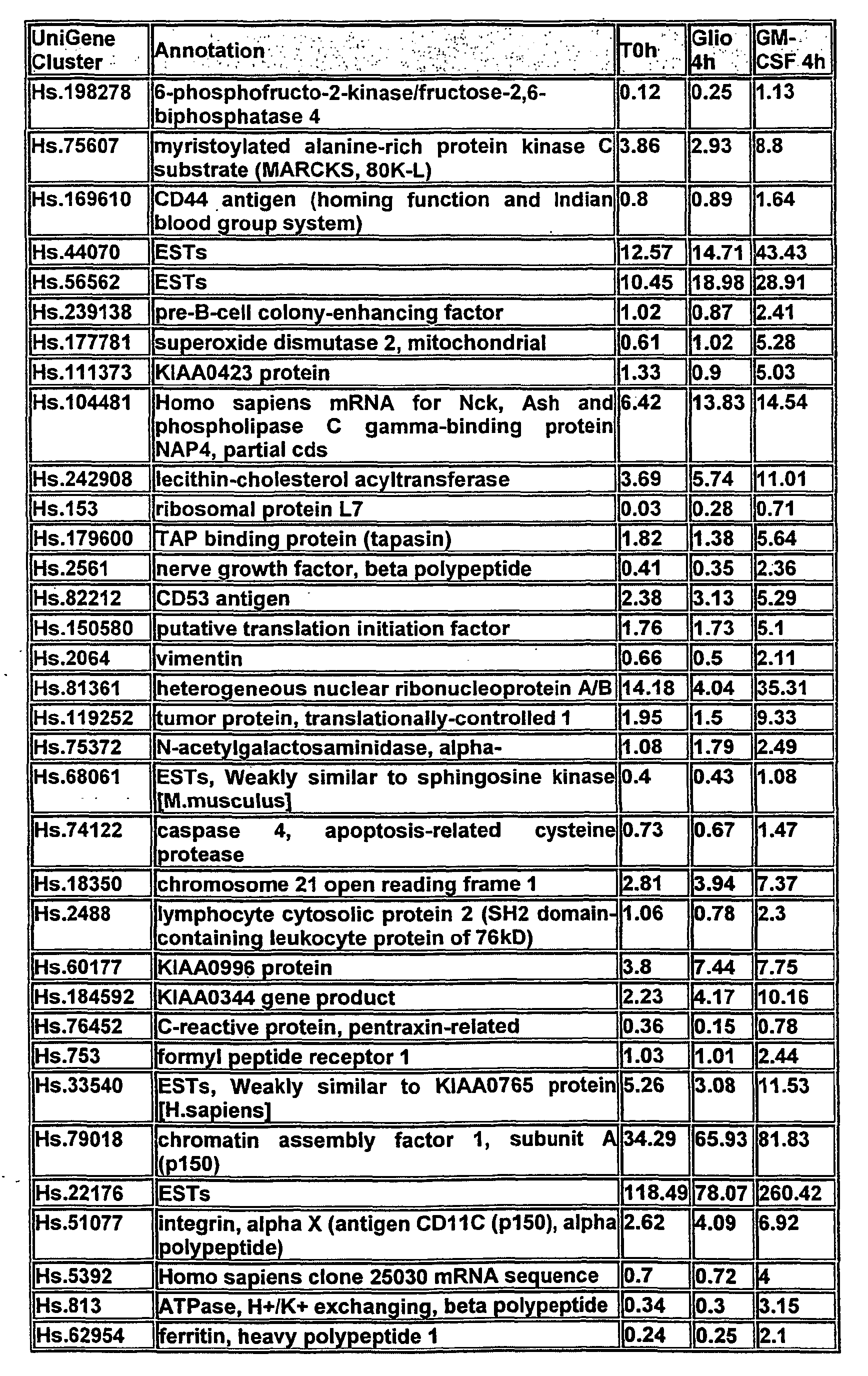

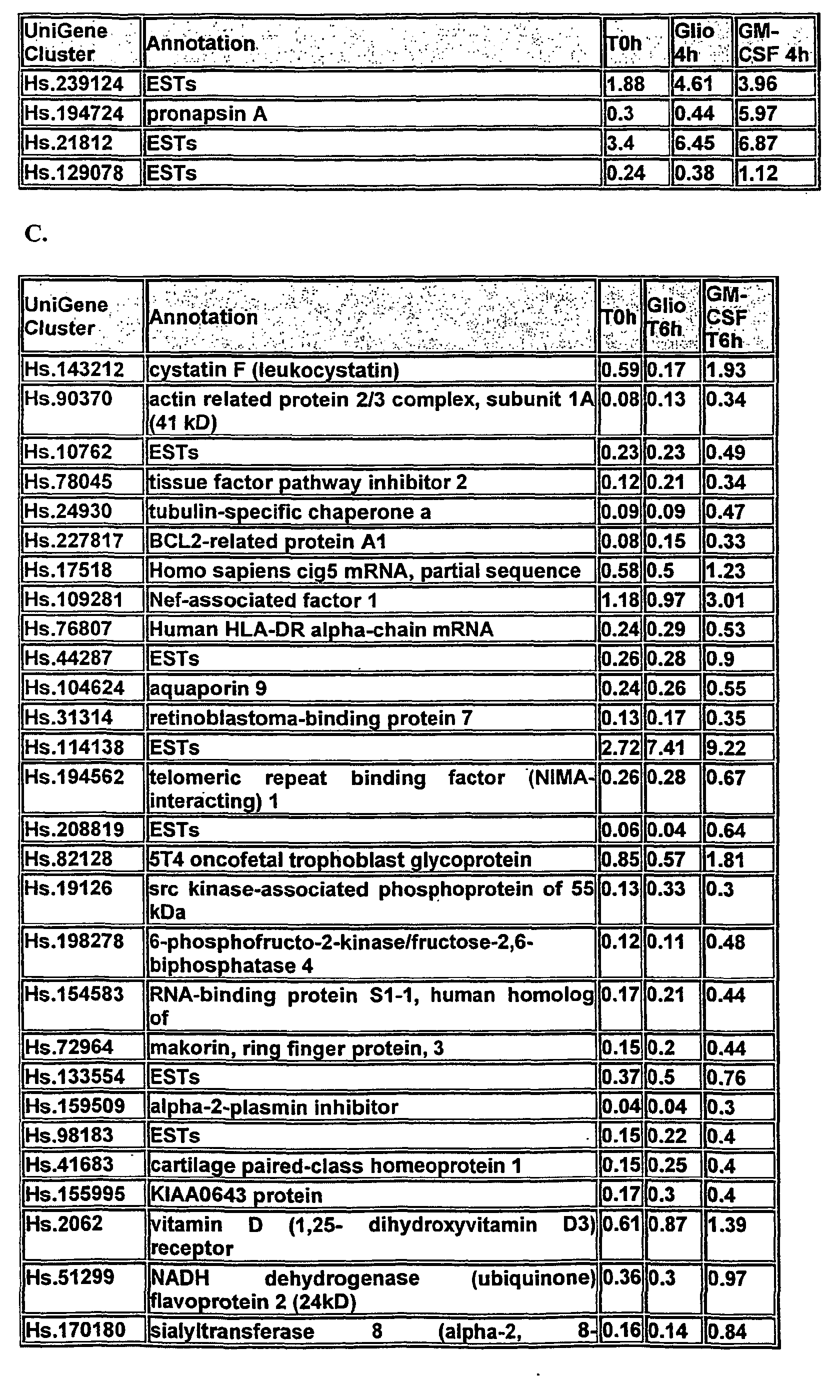

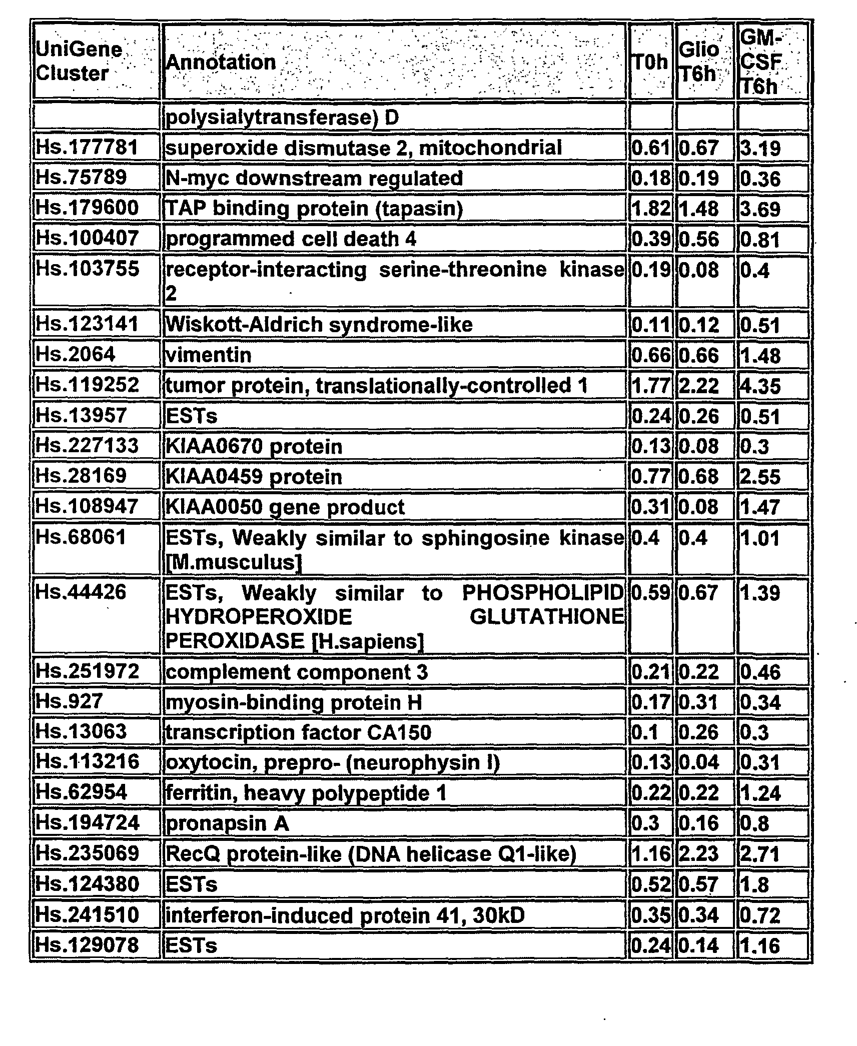

## Claims

1. A method for identifying in-vitro a gene product which modulates the transition of a cell between a non-apoptotic state and an apoptotic state, comprising the steps of:
(a) exposing the cell to an inhibitor of GM-CSF mediated inhibition of apoptosis; and
(b) exposing the cell to one or more agents which increase tyrosine phosphorylation; and
(c) placing the cell in conditions which permit it to undergo spontaneous apoptosis; and
(d) monitoring the level(s) of expression of the one or more gene products in the cell; and
(e) identifying gene product(s) whose expression has been increased, decreased or modified as a result of performing steps (a) to (d).

2. A method according to claim 1, further comprising the step of determining the level(s) of expression of one or more gene product(s) in a cell to establish a reference expression level;

3. A method according to claim 1 or claim 2 wherein one or more agent/s which increases tyrosine phosphorylation is selected from the group consisting of: phenylarsine oxide (PAO), Genestein.

4. A method according to any preceding claim wherein step (b); that is, exposing the cell to one or more agent/s which increases tyrosine phosphorylation is substituted by the step of; exposing the cell to GM-CSF.

5. A method according to any preceding claim wherein one or more inhibitor in step (a) is Gliotoxin.

6. A method according to claims 1 to 4 wherein one or more inhibitors in step (a) is an agent which increases the level ofNFkB within a cell.

7. A method according to claim 6 wherein one or more agents is any one of: LPS, TNFα, p60 component/s ofNFkB, p50 component/s ofNFkB.

8. A method according to claims 1 to 4 wherein the inhibitor in step (a) is one or more agent/s which inhibits the functions of NADPH oxidase.

9. A method according to claim 8 wherein one or more agents is any one of: phenylarsine oxide, diphenylene iodonium.

10. A method according to any preceding claim wherein the expression levels are determined by assessing polypeptide production.

11. A method according to any one of claims 1 to 9, wherein expression levels are determined by assessing polypeptide post-translational modification.

12. A method according to any one of claims 1 to 9, wherein expression levels are determined by assaying gene-transcription.

13. A method according to any preceding claim, wherein the cell is selected from the group consisting of neutrophils, cells with neutrophil characteristics such as HL60 cells, and HeLA cells.

14. A method according to claim 13 wherein the cell is cultured in the presence of an inhibitor of GM-CSF mediated inhibition of apoptosis.

15. A method according to any preceding claim wherein the onset of apoptosis is monitored by morphological analysis, externalisation of membrane phospholipid phosphatidyl serine or caspase activation analysis.

16. A method according to claim 12, wherein the expression levels of a plurality of gene product/s are determined by hybridisation of one or more mRNA populations to a set of polynucleotides arrayed on to a substrate.

17. A method of according to claim 10 or claim 11 wherein the expression levels of a plurality of gene product/s are determined by 2D-polyacrylamide gel electrophoresis of one or more polypeptide populations.

18. A method according to any preceding claim wherein the expression levels of the gene product/s are determined by analysis of global gene expression patterns.

19. A method according to claim 18 wherein global gene expression is analysed using microarray or SSH.

20. The use of gliotoxin to inhibit in-vitro the GM-CSF mediated inhibition of apoptosis.

## Patentansprüche

1. Verfahren zum Identifizieren eines Genprodukts, welches den Übergang einer Zelle zwischen einem nicht apoptotischen Zustand und einem apoptotischen Zustand moduliert, *in vitro,* wobei das Verfahren die folgenden Stufen umfaßt:
(a) Aussetzen der Zelle an einen Inhibitor von durch GM-CSF vermittelter Inhibition von Apoptose und
(b) Aussetzen der Zelle an ein oder mehrere Mittel, das bzw. die die Tyrosinphosphorylierung steigert bzw. steigern, und
(c) Einbringen der Zelle in Bedingungen, die es ihr erlauben, eine spontane Apoptose zu durchlaufen, und
(d) Überwachen der Expressionsmenge(n) des einen Genprodukts oder der mehreren Genprodukte in der Zelle und
(e) Identifizieren eines Genprodukts (von Genprodukten), dessen (deren) Expression infolge der Ausführung der Stufen (a) bis (d) gesteigert, verringert oder modifiziert wurde.

2. Verfahren nach Anspruch 1, welches weiterhin die Stufe umfaßt, bei der man die Expressionsmenge(n) des einen Genprodukts oder der mehreren Genprodukte in einer Zelle bestimmt, um eine Referenzexpressionsmenge festzulegen.

3. Verfahren nach Anspruch 1 oder Anspruch 2, wobei das eine oder die mehreren Mittel, das bzw. die die Tyrosinphosphorylierung steigert bzw. steigern, aus der Gruppe ausgewählt ist bzw. sind, bestehend aus: Phenylarsinoxid (PAO), Genestein.

4. Verfahren nach einem der vorangegangenen Ansprüche, wobei Stufe (b), d.h. das Aussetzen der Zelle an ein oder mehrere Mittel, das bzw. die die Tyrosinphosphorylierung steigert bzw. steigern, durch die Stufe ersetzt wird, bei der die Zelle an GM-CSF ausgesetzt wird.

5. Verfahren nach einem der vorangegangenen Ansprüche, wobei ein Inhibitor oder mehrere Inhibitoren in Stufe (a) Gliotoxin ist bzw. sind.

6. Verfahren nach einem der Ansprüche 1 bis 4, wobei ein Inhibitor oder mehrere Inhibitoren in Stufe (a) ein Mittel ist bzw. sind, welches die Menge an NFkB innerhalb einer Zelle erhöht.

7. Verfahren nach Anspruch 6, wobei ein oder mehrere Mittel irgendeines der folgenden ist bzw. sind: LPS, TNFα, (eine) p60-Komponente/n von NFkB, (eine) p50-Komponente/n von NFkB.

8. Verfahren nach einem der Ansprüche 1 bis 4, wobei der Inhibitor in Stufe (a) ein oder mehrere Mittel ist bzw. sind, welches bzw. welche die Funktionen von NADPH-Oxidase hemmt bzw. hemmen.

9. Verfahren nach Anspruch 8, wobei ein oder mehrere Mittel irgendeines der folgenden ist bzw. sind: Phenylarsinoxid, Diphenyleniodonium.

10. Verfahren nach einem der vorangegangenen Ansprüche, wobei die Expressionsmengen durch Feststellen der Polypeptidherstellung bestimmt werden.

11. Verfahren nach einem der Ansprüche 1 bis 9, wobei die Expressionsmengen durch Feststellen der posttranslationalen Polypeptidmodifikation bestimmt werden.

12. Verfahren nach einem der Ansprüche 1 bis 9, wobei die Expressionsmengen durch Untersuchen der Gentranskription bestimmt werden.

13. Verfahren nach einem der vorangegangenen Ansprüche, wobei die Zelle aus der Gruppe ausgewählt ist, bestehend aus Neutrophilen, Zellen mit neutrophilen Eigenschaften, wie HL60-Zellen, und HeLa-Zellen.

14. Verfahren nach Anspruch 13, wobei die Zelle in Gegenwart eines Inhibitors von durch GM-CSF vermittelter Inhibition von Apoptose kultiviert wird.

15. Verfahren nach einem der vorangegangenen Ansprüche, wobei der Beginn von Apoptose durch morphologische Analyse, Externalisierung von Membranphospholipidphosphatidylserin oder Caspaseaktivierungsanalyse überwacht wird.

16. Verfahren nach Anspruch 12, wobei die Expressionsmengen einer Vielzahl von Genprodukten durch Hybridisieren einer oder mehrerer mRNA-Populationen an einen Satz von auf einem Substrat angeordneten Polynukleotiden bestimmt werden.

17. Verfahren nach Anspruch 10 oder Anspruch 11, wobei die Expressionsmengen einer Vielzahl von Genprodukten durch 2D-Polyacrylamidgelelektroforese einer oder mehrerer Polypeptidpopulationen bestimmt werden.

18. Verfahren nach einem der vorangegangenen Ansprüche, wobei die Expressionsmengen der Genprodukte durch Analyse der globalen Genexpressionsmuster bestimmt werden.

19. Verfahren nach Anspruch 18, wobei die globale Genexpression unter Verwendung von Mikroarrays oder SSH analysiert wird.

20. Verwendung von Gliotoxin zur Hemmung von durch GM-CSF vermittelter Inhibition von Apoptose *in vitro.*

## Revendications

1. Procédé pour identifier *in vitro* un produit génique qui module la transition d'une cellule entre un état non apoptotique et un état apoptotique, comprenant les étapes suivantes :
(a) exposer la cellule à un inhibiteur d'inhibition d'apoptose médiée par GM-CSF ; et
(b) exposer la cellule à un ou plusieurs agents qui augmentent la phosphorylation de la tyrosine ; et
(c) placer la cellule dans des conditions qui lui permettent de subir une apoptose spontanée ; et
(d) contrôler le ou les taux d'expression d'un ou plusieurs des produits géniques dans la cellule ; et
(e) identifier un ou plusieurs produits géniques dont l'expression a été accrue, réduite ou modifiée en conséquence de l'exécution des étapes (a) à (d).

2. Procédé selon la revendication 1, comprenant de plus l'étape consistant à déterminer le ou les taux d'expression d'un ou plusieurs produits géniques dans une cellule pour établir un taux d'expression de référence.

3. Procédé selon la revendication 1 ou la revendication 2, dans lequel un ou plusieurs agents qui augmentent la phosphorylation de la tyrosine sont choisis dans le groupe formé par : l'oxyde de phénylarsine (PAO), la génestéine.

4. Procédé selon l'une quelconque des revendications précédentes, dans lequel l'étape (b), c'est-à-dire l'exposition de la cellule à un ou plusieurs agents qui augmentent la phosphorylation de la tyrosine, est remplacée par l'étape consistant à exposer la cellule à GM-CSF.

5. Procédé selon l'une quelconque des revendications précédentes, dans lequel un ou plusieurs inhibiteurs dans l'étape (a) sont la gliotoxine.

6. Procédé selon les revendications 1 à 4, dans lequel un ou plusieurs inhibiteurs dans l'étape (a) sont un agent qui augmente le taux de NFkB dans une cellule.

7. Procédé selon la revendication 6, dans lequel un ou plusieurs agents sont l'un quelconque de : LPS, TNFα, composant(s) p60 de NFkB, composant(s) de p50 de NFkB.

8. Procédé selon les revendications 1 à 4, dans lequel l'inhibiteur dans l'étape (a) est un ou plusieurs agents qui inhibent les fonctions de la NADPH-oxydase.

9. Procédé selon la revendication 8, dans lequel un ou plusieurs agents sont l'un quelconque de : oxyde de phénylarsine, diphénylène-iodonium.

10. Procédé selon l'une quelconque des revendications précédentes, dans lequel les taux d'expression sont déterminés en évaluant la production de polypeptides.

11. Procédé selon l'une quelconque des revendications 1 à 9, dans lequel les taux d'expression sont déterminés en évaluant la modification post-traductionnelle de polypeptides.

12. Procédé selon l'une quelconque des revendications 1 à 9, dans lequel les taux d'expression sont déterminés par mesure de transcription génique.

13. Procédé selon l'une quelconque des revendications précédentes, dans lequel la cellule est choisie dans le groupe formé par les neutrophiles, les cellules ayant des caractéristiques neutrophiles telles que les cellules HL60, et les cellules HeLA.

14. Procédé selon la revendication 13, dans lequel la cellule est cultivée en présence d'un inhibiteur d'inhibition d'apoptose médiée par GM-CSF.

15. Procédé selon l'une quelconque des revendications précédentes, dans lequel l'apparition d'apoptose est contrôlée par analyse morphologique, externalisation de phosphotidylsérine phospholipidique membranaire ou analyse d'activation des caspases.

16. Procédé selon la revendication 12, dans lequel les taux d'expression de plusieurs des produits géniques sont déterminés par hybridation d'une ou plusieurs populations d'ARNm avec un jeu de polynucléotides rangés sur un substrat.

17. Procédé selon la revendication 10 ou la revendication 11, dans lequel les taux d'expression de plusieurs produits géniques sont déterminés par électrophorèse 2D sur gel de polyacrylamide d'une ou plusieurs populations de polypeptides.

18. Procédé selon l'une quelconque des revendications précédentes, dans lequel les taux d'expression des produits géniques sont déterminés par analyse de profils d'expression génique globale.

19. Procédé selon la revendication 18, dans lequel l'expression génique globale est analysée par utilisation de microarray ou de SSH.

20. Utilisation de gliotoxine pour inhiber *in vitro* l'inhibition d'apoptose médiée par GM-CSF.
